# EUROPEAN PATENT SPECIFICATION

(11) **EP 3 044 594 B1**
(45) Date of publication and mention of the grant of the patent: **29.08.2018**
(21) Application number: 14843544.9
(22) Date of filing: 11.09.2014
(51) Int. Cl.: G01N 33/564, C12Q 1/6883

(54) **IDENTIFICATION OF NOVEL BIOMARKERS OF FLARES OF SYSTEMIC LUPUS ERYTHEMATOSUS**
IDENTIFIKATION NEUARTIGER BIOMARKER FÜR DAS AUFFLAMMEN EINES SYSTEMISCHEM LUPUS ERYTHEMATODES
IDENTIFICATION DE NOUVEAUX BIOMARQUEURS DE POUSSÉES DE LUPUS ÉRYTHÉMATEUX DISSÉMINÉ

(30) Priority: 11.09.2013 SG 201306892
(43) Date of publication of application: 20.07.2016
(73) Proprietor: Singapore Health Services Pte Ltd, Singapore 168753 (SG)
(72) Inventor: FONG, Kok Yong, Singapore 169856 (SG); THUMBOO, Julian, Singapore 169856 (SG); THIO, Szu-tien, Singapore 169856 (SG); TONG, Hak Tien, Louis-Marie Grignion, Singapore 168751 (SG); ZHOU, Lei, Singapore 168751 (SG)
(74) Representative: Bailey, Jennifer Ann
(86) International application number: PCT/SG2014/000428
(87) International publication number: WO 2015/038069

(56) References cited:
- WO-A1-2007/126391
- WO-A1-2007/126391
- WO-A1-2008/032868
- WO-A1-2011/127219
- WO-A2-2012/032345
- US-A1- 2005 142 616
- RECORD J.L. ET AL.: "High prevalence of myositis in a southeastern United States pediatric systemic lupus erythematosus cohort", PED. RHEUMATOL., vol. 9, no. 1, 9 August 2011 (2011-08-09), page 20, XP021109198,
- HU S ET AL.: 'Preclinical validation of salivary biomarkers for primary Sjogren's syndrome' ARTHRITIS CARE RES vol. 62, no. 11, 2010, pages 1633 - 1638, XP055324519
- MICHELA CAPELLO ET AL.: 'a-enolase: a promising therapeutic and diagnostic tumortarget' FEBS JOURNAL vol. 278, 2011, pages 1064 - 1074, XP055292536
- ASHIZAWA TADASHI ET AL.: 'Expression of deleted malignant brain tumor 1 (DMBT1) in cultured tumor cell lines' PROCEEDINGS OF THE JAPANESE CANCER ASSOCIATION vol. 66 TH, 2007, page 174, XP008183430

## Description

### FIELD OF INVENTION

The present invention relates generally to methods for diagnosing or monitoring the flare status of subjects with Systemic Lupus Erythematosus (SLE). More particularly, the present invention relates to screening samples from subjects for particular protein biomarkers which have efficacy in identifying or predicting a flare.

### BACKGROUND

The prevalence of Systemic Lupus Erythematosus (SLE) ranges from 40/100,000 cases for Northern Europeans to 200/100,000 cases in Blacks. There are more than 250,000 patients with SLE in the United States. The 15-year survival rate is 80-85% and the chance of dying by 35 years of age from lupus or infection is 1 in 6 patients. WO 2012/032345 A2 discloses a method and kit for determining SLE-associated disease state in an individual, in particular the flare status, comprising measuring the presence and/or amount of one or more biomarker(s) selected from a list of 58 biomarkers. Currently available biomarkers for SLE flares (dsDNA, C3, C4) are useful in detecting flares in 60-70% of SLE patients, but do not detect flares in a substantial number of patients, including those with major organ involvement e.g. lupus nephritis. They are therefore of limited value for routine clinical use. These biomarkers also require blood draws which are associated with discomfort for patients. Frequent, regular monitoring of biomarkers is important in predicting incipient flares in SLE. There is therefore an unmet need and tear biomarkers, if validated, may fulfill this role as the collection is non-invasive and more convenient in comparison with blood draws. As some tear proteins are involved in immune system functioning, the study of potential biomarkers in tears is a novel area of research in autoimmune disease.

Flares occur in approximately 80% of patients during the course of their disease [Petri M, et al., Am J Med 1991; 91: 345-54], and generally require the introduction or increase in dose of a variety of potentially toxic therapies. The morbidity and mortality associated with flares can be substantial, and is related to organ damage resulting from active SLE per se and the adverse effects of corticosteroids and immunosuppressive drugs [Abu-Sharaka M, et al., J Rheumatol 1995; 22: 1259-64]. In SLE patients with nephritis, for example, doubling of serum creatinine was 7 times more likely in patients with a renal flare, and 27 times more likely in severe renal flares manifesting as nephritic syndrome [Petri M, et al., Arthritis Rheum 1991; Aug 34(8): 937-44]. Flares also result in significant mortality,
accounting for 26% of deaths within 5 years of diagnosis and 10% of deaths more than 5 years after diagnosis in one series [Abu-Sharaka M, et al., J Rheumatol 1995; 22: 1259-64]. Flares of disease activity often necessitate the use of corticosteroids and/ or immunosuppressives. Corticosteroid use may lead to osteoporosis, infections (at times fatal) and avascular necrosis of bone [Ehrenstein MR, et al., Br J Rheumatol 1995; 34(3): 257-60]. Immunosuppressive therapy is also associated with significant morbidity and mortality. For example, 27% of 127 SLE patients treated with cyclophosphamide in Singapore developed major infections, with 4 patients perishing from these infections [Mirzayan MJ, et al., Rheumatology (Oxford) 2000; 39(12): 1316-9].

Flares of SLE disease activity (a major source of morbidity/ mortality) are inadequately predicted by existing biomarkers. As some tear and saliva proteins are involved in immune system functioning, the study of potential biomarkers in tears and saliva is a novel area of research in autoimmune disease. We used iTRAQ with nano LC-MS/MS [Zhou L, et al., J Proteome Res 2009; 8: 4889-905] to monitor changes in the levels of various potential protein biomarkers in serial tear and saliva specimens from SLE patients.

### SUMMARY OF THE INVENTION

The expression signatures identified in this study had sufficient diagnostic efficacy for development into tear- and saliva-based biomarkers for diagnosing or monitoring the flare status in subjects with SLE. Accordingly, in a first aspect, the present invention provides a method of diagnosing or monitoring the flare status of SLE in a subject, comprising screening a fluid test sample from the subject for the presence of at least one biomarker differentially expressed in a flare state compared to a reference sample, wherein the at least one biomarker is selected from the group comprising Enolase 1 (ENO1), ), Deleted in Brain Tumour 1 (DMBT1), Lactate dehydrogenase A (LDHA), Lactate dehydrogenase B (LDHB), Heat Shock 70kDa Protein 1B (HSPA1B) and Heat Shock 27kDa Protein 1 (HSPB1); and diagnosing or monitoring the status of SLE in the subject based on the expression of the at least one biomarker.

Embodiments of the invention are provided by the dependent claims.

In a related aspect, there is described a method of analysing a fluid test sample from a subject, comprising a step of determining the level of at least one biomarker in the test sample and comparing said level to that in a reference sample, wherein the at least one biomarker is selected from the group comprising Enolase 1 (ENO1), Deleted in Brain Tumour 1 (DMBT1), Lactate dehydrogenase A (LDHA) or Lactate dehydrogenase B (LDHB) and Heat Shock 70kDa Protein 1B (HSPA1B) and Heat Shock 27kDa Protein 1 (HSPB1). In a preferred embodiment of the invention, the at least one biomarker comprises DMBT1.

There is also provided a method of diagnosing or monitoring the flare status of SLE in a subject, comprising screening a fluid test sample from the subject for the presence of at least one biomarker differentially expressed in a flare state compared to a reference sample, wherein the at least one biomarker is selected from the group consisting of ENO1, DMBT1, LDHA, LDHB, HSPA1B and HSPB1; and
diagnosing or monitoring the status of SLE in the subject, based on expression of the at least one biomarker.

In a preferred embodiment of the invention, the test sample is selected from the group comprising tear, saliva, blood and urine.

In a preferred embodiment of the invention, the reference sample represents the biomarker expression profile of a normal subject or an SLE subject in a quiescent or flare state.

In another preferred embodiment of the invention, the reference sample represents the biomarker expression profile of an SLE subject in a quiescent state.

In another preferred embodiment of the invention, the status of SLE in a subject is a state of incipient flare of disease activity.

In another preferred embodiment of the invention, the expression of the at least one biomarker is detected using tandem Mass Spectrometry, using antibodies directed to said biomarker in immunoassay.

In another related aspect of the invention, the flare status of SLE in a subject comprises response to a treatment administered to the subject.

There is also described a SLE diagnostic or monitoring kit comprising at least one probe which specifically binds to at least one biomarker selected from the group consisting of ENO1, DMBT1, LDHA, LDHB, HSPA1B and HSPB1.

### BRIEF DESCRIPTION OF THE FIGURES

Figure 1: Flow chart of study subjects and study enrollment.

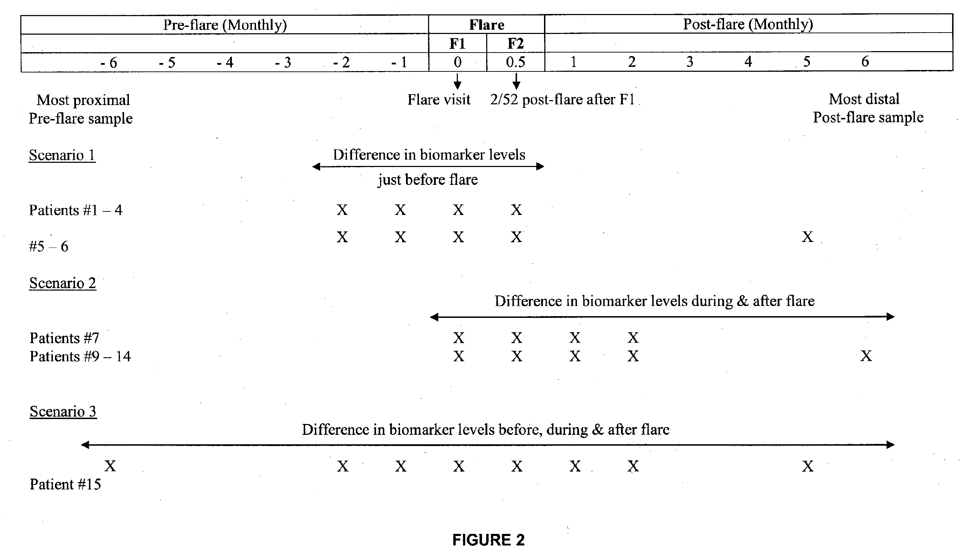

Figure 2: Sampling of tear specimens for subsets of patients.
Figure 3: Experimental design for iTRAQ proteomics.
Figure 4: Ratio Proteomic Profile: Flare vs non-Flare average from 8 patients.
Figure 5: Log Ratio Proteomic Profile: Flare vs non-Flare average from 8 patients.
Figure 6: Numbers 1-16 in (A) and (B) indicate 16 flare states (from 8 patients, each patient has 2 flare states). Numbers 1-8 in (C) and (D) indicate 8 non-flare states (from 8 patients, each patient has 1 non-flare state). Dark: protein level indicates the flare state. Light: protein level fails to indicate the flare state. (A): Combination of DMBT1 and LDHB, ratio > 1.5 or < 0.67, 15/16 (93.8%) correct.; (B):
   Combination of DMBT1 and ENO1, ratio > 1.5 or < 0.67, 16/16 (100%) correct; (C): Combination of DMBT1 and LDHB, ratio < 1.5 or > 0.67, 8/8 (100%) correct whereas if use single biomarker of DMBT1 resulted in 6/8 (75%) correct and LDHB resulted in 4/8 (50%) correct; (D): Combination of DMBT1 and ENO1, ratio < 1.5 or > 0.67, 8/8 (100%) correct whereas if use single biomarker of either DMBT1 or ENO1 resulted in 6/8 (75%) correct.

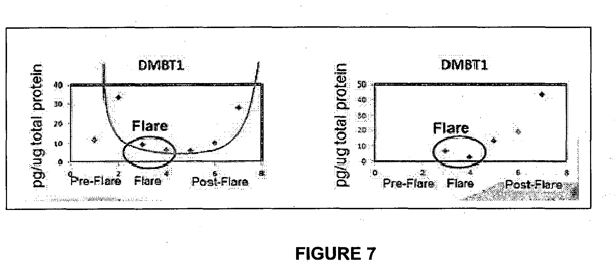

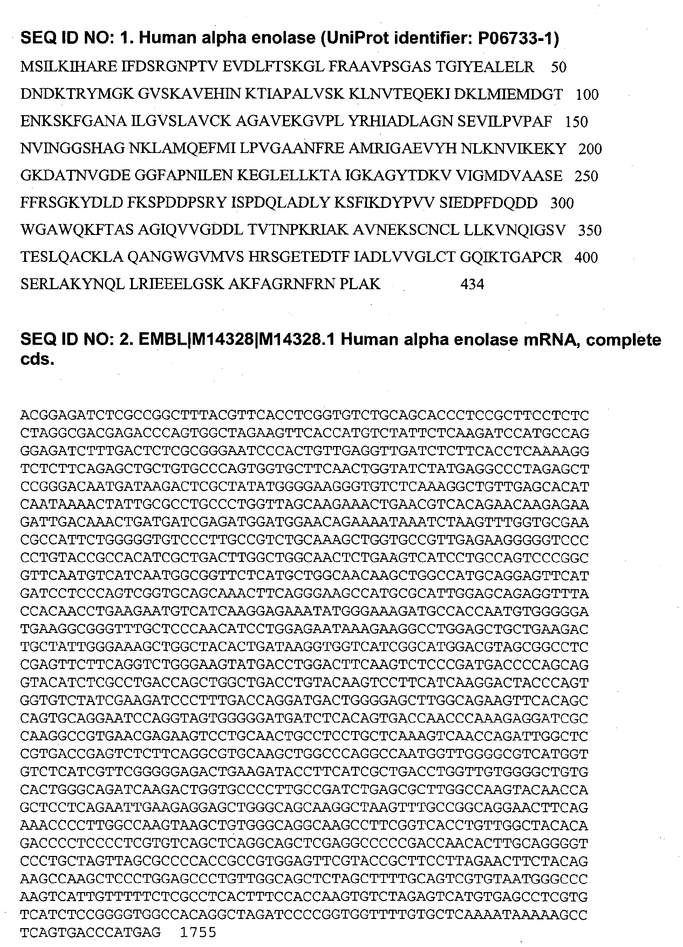

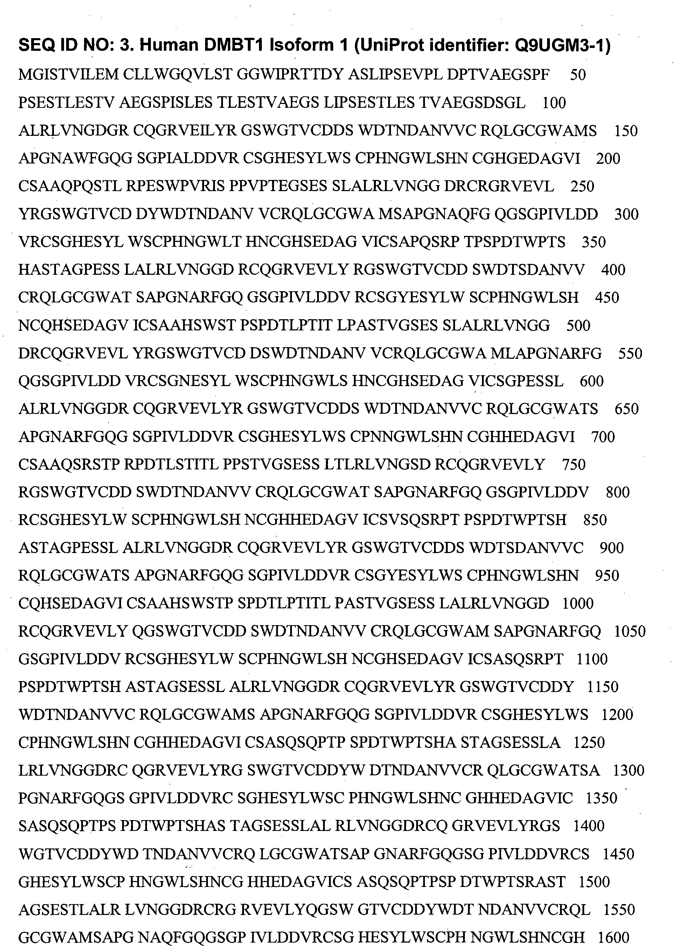

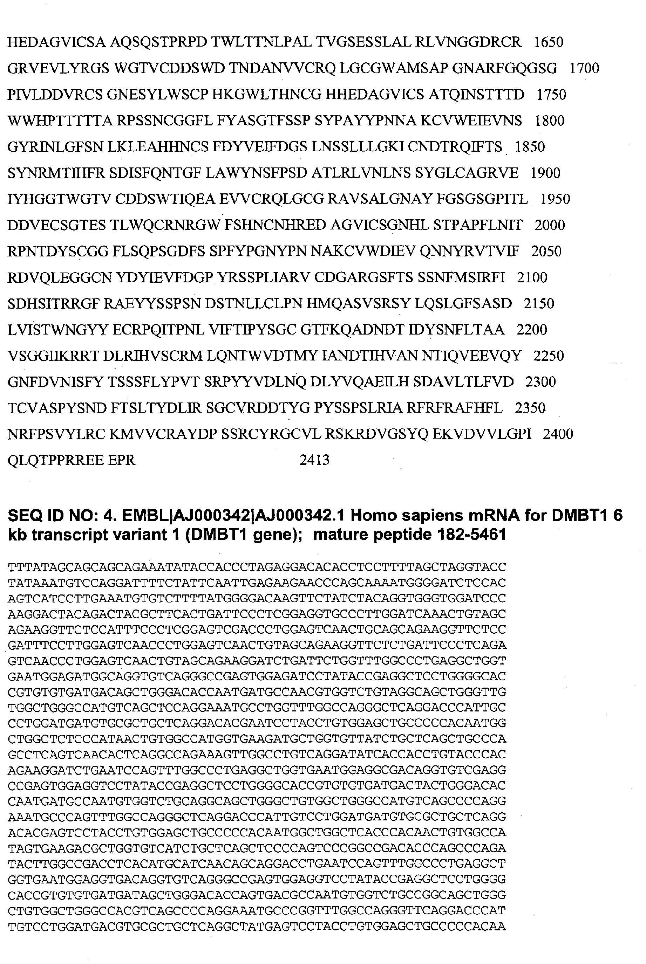

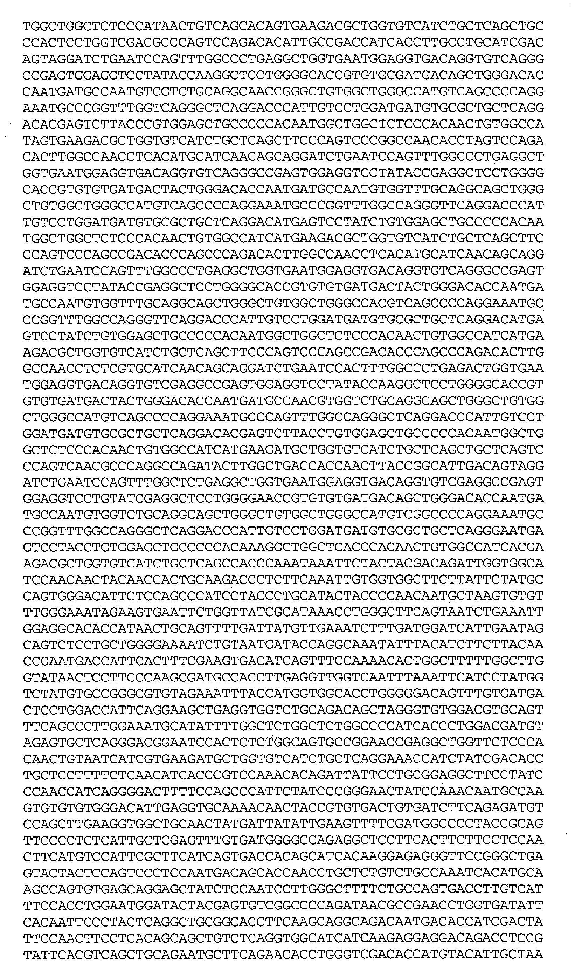

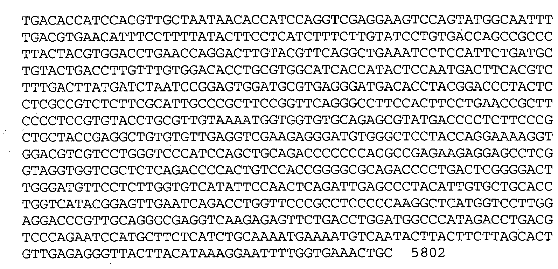

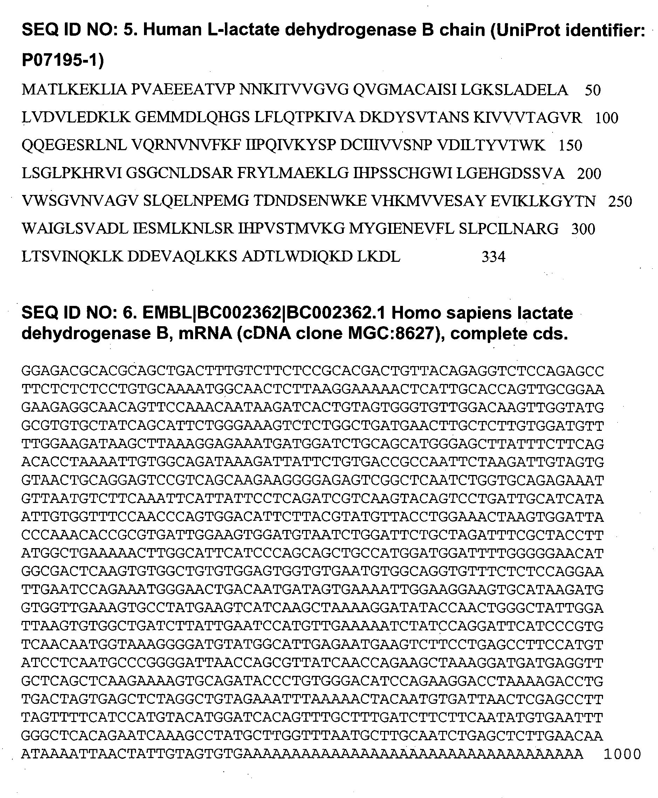

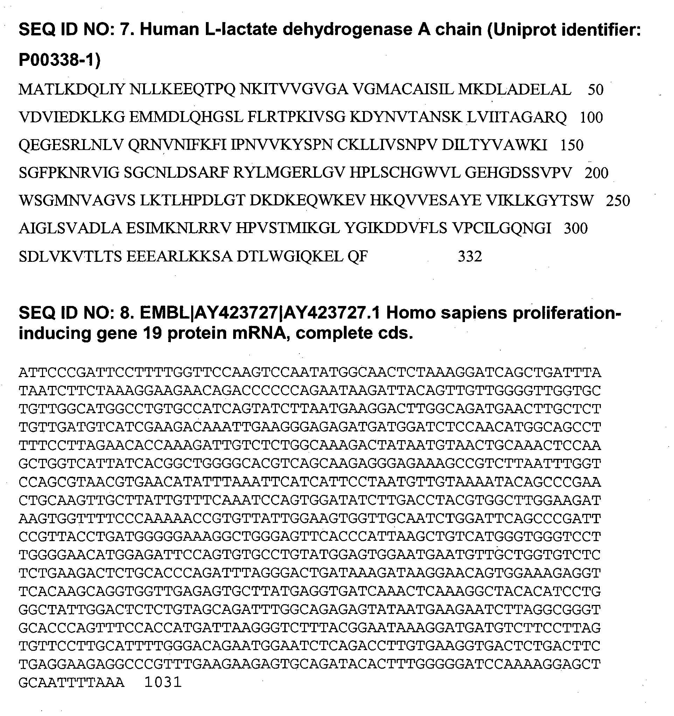

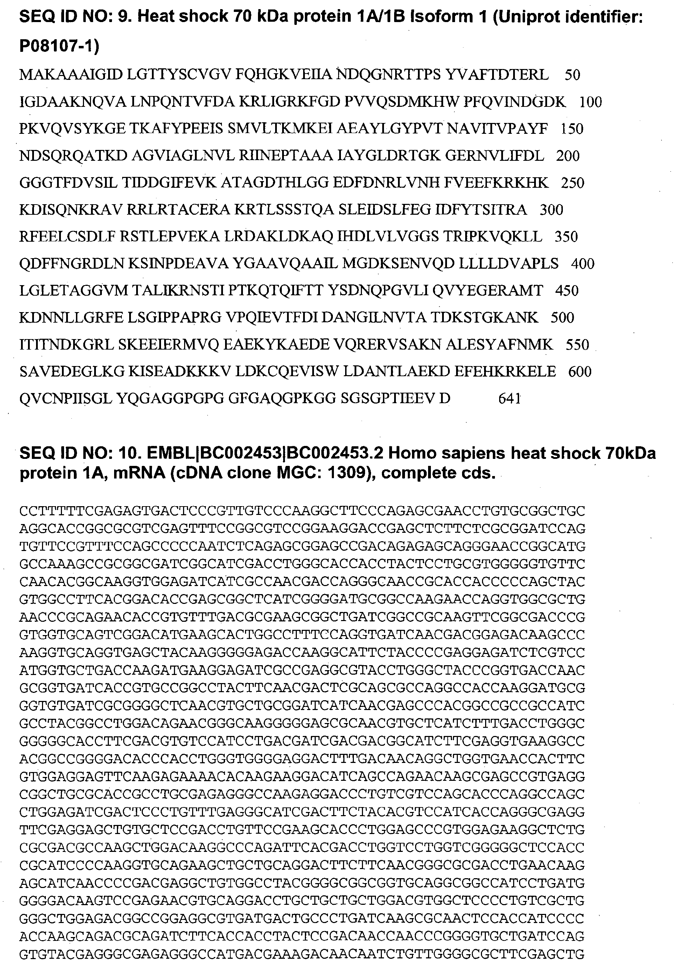

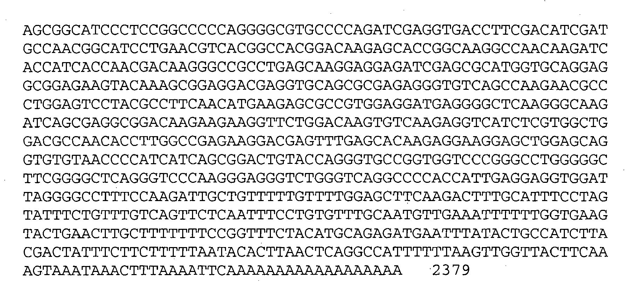

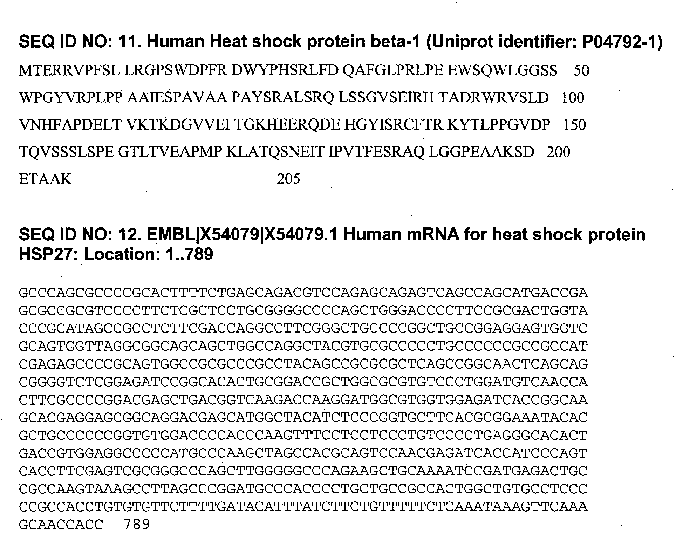

Figure 7: Tear DMBT1 levels in two individual patients with SLE in pre-flare, flare and post-flare states. (1 and 2: pre-Flare, 3 and 4: Flare, 5, 6 and 7: post-Flare).

### DETAILED DESCRIPTION

Although various body fluids may be tested for the presence of flare biomarkers, the collection of tears, urine and saliva is relatively non-invasive as compared to withdrawal of blood. Such tear, urine and saliva sampling would allow for more regular collection of specimens, even by the patient themselves if a suitable sampling and testing kit were available. If a patient could test their own tears, urine or saliva for biomarkers indicative of a flare state, they would have a stronger incentive to follow up with further analysis by seeing a doctor for assessment of SLE flare status. Frequent regular monitoring of biomarkers is likely to be important in predicting incipient flares, as the levels of such biomarkers may rise or fall relatively proximately to a flare.

### Definitions

Certain terms employed in the specification, examples and appended claims are collected here for convenience.

An SLE flare is defined herein as either SLEDAI score ≥ 4 or physician assessed flares. The rationale for including physician assessed flares is that the SLEDAI does not cover some rarer manifestations of flares, e.g. gut vasculitis, new onset of peripheral neuropathy.

As used herein, 'incipient flare' means the sub-clinical, beginning, early or emerging stages of a flare.

As used herein, 'quiescent state' means the subject is not experiencing a clinical flare, in the presence or absence of serological indications. We do note that 10% of SLE patients are "clinically quiescent, biochemically active" (i.e. no clinical flare though existing biomarkers suggest the presence of a flare) and another 10% of SLE patients are "clinically active, biochemically quiescent" (i.e. clinical flare though existing biomarkers do not suggest the presence of a flare) and that the proposed patent can help to clarify the clinical state of these patients.

As used herein, 'biomarker' refers to a protein molecule or nucleic acid which is differentially expressed in the tears and/or saliva during the quiescent state compared to flare state in SLE patients. According to the invention one or more biomarkers may be selected from Enolase 1 (ENO1), Deleted in Brain Tumour 1 (DMBT1), Lactate dehydrogenase A (LDHA), Lactate dehydrogenase B (LDHB), Heat Shock 70kDa Protein 1B (HSPA1B) and Heat Shock 27kDa Protein 1 (HSPB1).

A representative amino acid sequence of human ENO1 is shown in SEQ ID NO: 1, and nucleic acid sequence shown in SEQ ID NO: 2. A representative amino acid sequence of human DMBT1 is shown in SEQ ID NO: 3, and nucleic acid sequence shown in SEQ ID NO: 4. A representative amino acid sequence of human LDHA is shown in SEQ ID NO: 5, and nucleic acid sequence shown in SEQ ID NO: 6. A representative amino acid sequence of human LDHB is shown in SEQ ID NO: 7, and nucleic acid sequence shown in SEQ ID NO: 8. A representative amino acid sequence of human HSPA1B is shown in SEQ ID NO: 9, and nucleic acid sequence shown in SEQ ID NO: 10. A representative amino acid sequence of human HSPB1 is shown in SEQ ID NO: 11 and nucleic acid sequence shown in SEQ ID NO: 12.

An antibody is any immunoglobulin, including antibodies and fragments thereof that bind to a specific epitope. An antibody for use in the invention may be prepared against an isolated or recombinant form of a biomarker protein of the invention. Such antibodies include, but are not limited to polyclonal, monoclonal, chimeric, humanised, single chain, Fab, Fab', F(ab)' fragments and/or F(v) portions of the whole antibody which are capable of binding to a biomarker to enable its identification and quantitation. The polypeptide or oligopeptide used to immunize an animal (e.g., a mouse, a rat, or a rabbit) can be derived from the translation of RNA, an expression construct such as a plasmid, or synthesized chemically, and can be conjugated to a carrier protein if desired. Commonly used carriers that are chemically coupled to peptides include bovine serum albumin, thyroglobulin, and keyhole limpet hemocyanin (KLH). The coupled peptide is then used to immunize the animal. Suitable biomarker detection antibodies may also be commercially available.

The term "oligonucleotide," as used herein, refers to a nucleic acid sequence of at least about 6 nucleotides to 60 nucleotides, preferably about 15 to 30 nucleotides, and most preferably about 20 to 25 nucleotides, which can be used in PCR amplification or in a hybridization assay. As used herein, the term "oligonucleotide" is substantially equivalent to the terms "amplimers," "primers," "oligomers," and "probes," as these terms are commonly defined in the art.

The term "comprising" as used in the context of the invention refers to where the various components, ingredients, or steps, can be conjointly employed in practicing the present invention. Accordingly, the term "comprising" encompasses the more restrictive terms "consisting essentially of" and "consisting of." With the term "consisting essentially of" it is understood that the biomarker of the present invention "substantially" comprises the indicated protein as "essential" element. Additional markers may be included in the methods and kits of the invention.

One aspect of the present invention provides a method of diagnosing or monitoring the flare status of SLE in a subject, comprising screening a fluid test sample from the subject for the presence of at least one biomarker differentially expressed in a flare state compared to a reference sample, wherein the at least one biomarker is selected from the group comprising Enolase 1 (ENO1), ), Deleted in Brain Tumour 1 (DMBT1), Lactate dehydrogenase A (LDHA), Lactate dehydrogenase B (LDHB), Heat Shock 70kDa Protein 1B (HSPA1B) and Heat Shock 27kDa Protein 1 (HSPB1); and diagnosing or monitoring the status of SLE in the subject based on the expression of the at least one biomarker.

Embodiments of the invention are provided by the dependent claims.

A related aspect of the present invention describes a method of analysing a fluid test sample from a subject, comprising a step of determining the level of at least one biomarker in the test sample and comparing said level to that in a reference sample, wherein the at least one biomarker is selected from the group comprising Enolase 1 (ENO1), Deleted in Brain Tumour 1 (DMBT1), Lactate dehydrogenase A (LDHA) or Lactate dehydrogenase B (LDHB) and Heat Shock 70kDa Protein 1B (HSPA1B) and Heat Shock 27kDa Protein 1 (HSPB1).

In a preferred embodiment of the invention, the at least one biomarker comprises DMBT1. Preferably the at least one biomarker comprises DMBT1 and LDHB or DMBT1 and ENO1. The studies described herein show that these pairs of biomarkers increased the accuracy of diagnosis to 100%.

Preferably the subject is a human.

Preferably, the test sample and the reference sample isolated from the subject are fluid samples, such as from tears, saliva, blood or urine. More preferably the sample is a tear or saliva sample. Most preferably the sample is a tear sample.

There is also provided a method of diagnosing or monitoring the flare status of SLE in a subject, comprising screening a fluid test sample from the subject for the presence of at least one biomarker differentially expressed in a flare state compared to a reference sample, wherein the at least one biomarker is selected from the group consisting of ENO1, DMBT1, LDHA, LDHB, HSPA1B and HSPB1; and

diagnosing or monitoring the status of SLE in the subject based on expression of the at least one biomarker.

In a preferred embodiment of the invention, the reference sample represents the biomarker expression profile of a normal subject or an SLE subject in a quiescent or flare state.

In another preferred embodiment of the invention, the reference sample represents the biomarker expression profile of an SLE subject in a quiescent state.

In another preferred embodiment of the invention, the status of SLE in a subject is a status of incipient flare of disease activity.

In another preferred embodiment of the invention, the expression of the at least one biomarker is detected using tandem Mass Spectrometry, using antibodies directed to said biomarker or using a nucleic acid technology.

A suitable method and equipment to perform Mass Spectrometry is described in the examples herein.

Suitable antibodies may be generated using conventional methods, including animal immunization with an isolated or recombinant biomarker protein or antigenic fragment thereof. A suitable isolated or recombinant biomarker protein may have the sequence set forth in SEQ ID NO: 1, SEQ ID NO: 3, SEQ ID NO: 5, SEQ ID NO: 7, SEQ ID NO: 9 or SEQ ID NO: 11. Isoforms or splice variants of these proteins and biomarker proteins from other species which are homologous to the aforementioned biomarkers may also be suitable as antigens to generate antibodies for use according to the invention.

The biomarker proteins of the invention may be produced for immunization purposes synthetically or via expression constructs which encode them. An example of a suitable expression vector is the bacterial plasmid pGEX-4T-1, which encodes a fusion protein with a thrombin cleavage site and is available from GE Healthcare Bio-Sciences Corp, NJ, USA.

Alternatively, commercial antibodies to the said biomarkers may be used. For example, mouse anti human DMBT1 monoclonal antibody [HYB 213-01-02]; mouse anti-alpha-enolase (anti-ENO1) antibody, monoclonal [MA5-17627]; mouse anti-L-lactate dehydrogenase B chain antibody, monoclonal [MA5-17242]; mouse anti-L-lactate dehydrogenase A chain antibody, monoclonal [MA5-17247]; and mouse anti-Heat shock protein beta-1 antibody, monoclonal [G3.1] are available from Thermo Fisher Scientific, MA, USA.

In another preferred embodiment of the invention, the nucleic acid technology further comprises hybridization or amplification in a quantitative real-time polymerase chain reaction.

In another preferred embodiment of the invention, detecting expression of the at least one biomarker further comprises isolating RNA from a subject sample.

In another preferred embodiment of the invention, detecting expression comprises using at least one primer or probe set to detect the expression of each of the at least one biomarker. The at least one primer or probe set may be based on one or more of the nucleic acid sequences set forth in SEQ ID NO: 2, SEQ ID NO: 4, SEQ ID NO: 6, SEQ ID NO: 8, SEQ ID NO: 10, or SEQ ID NO: 12 or variants or homologues thereof.

The primers or probe sets may comprise a diagnostic kit.

In another related aspect of the invention, the status of SLE in a subject comprises response to a treatment administered to the subject. The treatment may be administration of one or more from the group comprising NSAIDS, corticosteroids, antimalarials (such as hydroxychloroquine) and immunosuppressive drugs.

Another related aspect of the invention provides a SLE flare diagnostic or monitoring kit comprising at least one probe which specifically binds to at least one biomarker selected from the group consisting of ENO1, DMBT1, LDHA, LDHB, HSPA1B and HSPB1.

The kit may comprise one or more antibodies specific to each of the at least one biomarker protein. The kit may also contain suitable solid supports and reagents for western blot, dot blot, ELISA, lateral flow assay (LFA) or any other immunoassay platform using various labeling techniques such as enzyme amplification, radioactivity or fluorescence. An advantage of using ELISA assay is that it is an established and sensitive technique to quantify analytes. ELISA methods are, for example, described in Thermo Scientific Pierce Assay Development Technical Handbook (thermoscientific.com/pierce), incorporated herein by reference. LFA is cheap and easy to use and provides results in 15-20 minutes. LFA is a well-known immunoassay platform, an example of which is described in Lee LG, et al., Biosensors 2013, 3, 360-373. Preferably the kit has components suitable for the detection of DMBT1 and ENO1, or DMBT1 and LDHB.

According to any aspect of the present invention, existing SLE biomarkers (e.g. dsDNA, complement levels) may additionally be tested concurrently or sequentially from blood samples and their results combined with the results of the present invention to increase the accuracy of detecting flares of SLE.

A person skilled in the art will appreciate that the present invention may be practiced without undue experimentation according to the methods given herein. The methods, techniques and chemicals are as described in the references given or from protocols in standard biotechnology and molecular biology text books, such as described in Sambrook and Russell, Molecular Cloning: A Laboratory Manual, Cold Springs Harbor Laboratory, New York (2001).

### EXAMPLES

We hypothesised that there are biomarkers in fluid samples of SLE patients which are indicative of flare status. We tested this hypothesis on tear samples by using mass spectroscopy to identify changes in the levels of various potential biomarkers in serial tear specimens from SLE patients.

### Study Design

In this IRB approved study, we prospectively studied 100 subjects with SLE selected from an already assembled cohort of well characterised SLE patients (n=350) in the Department of Rheumatology and Immunology, Singapore General Hospital. Subjects were assessed monthly for 12 months. Additionally, if a patient had a flare of SLE during this period, he/ she was reviewed 2 weeks after the flare.

### Experimental Approaches

All enrolled subjects had monthly assessments of:
- SLE disease activity measured using SLEDAI 2000, MEX-SLEDAI and Classic BILAG
- Presence / severity of dry eyes with monthly Schirmer's test
- ACR Damage Index is used to record any damage at the 1^{st} and last study visit
- Medication use including eye drops
- Information on infection or change in treatment including use of Traditional Chinese Medicine since the previous visit

### Inclusion and exclusion criteria:

Inclusion criteria were age 16 years and above, a diagnosis of SLE (fulfilling 4 of 11 American College of Rheumatology 1997 criteria); ability to give written informed consent. For patients 16 to 20 years, informed consent was obtained from the legally acceptable representative (including spouse, parent and guardian).

Exclusion criteria: Inability or refusal to give written informed consent.

### Cohort Characteristics

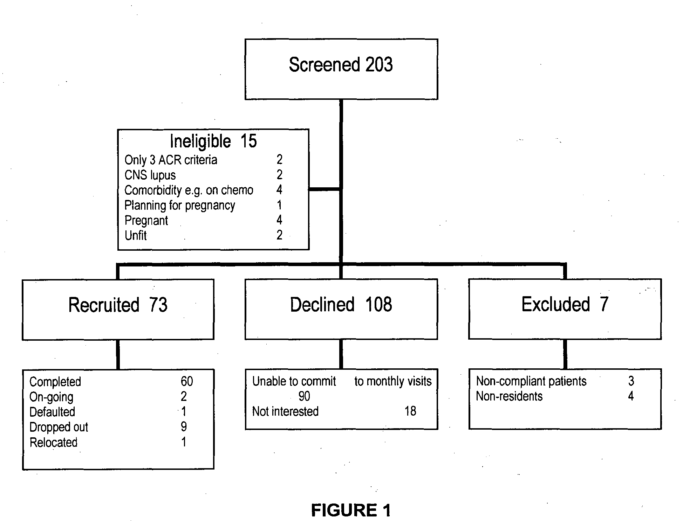

203 subjects were screened, 108 declined, 7 excluded and 15 were ineligible, leaving 73 subjects recruited (Figure 1).

Of 73 patients with SLE, 14 patients had flares in the study period. The demographic information of these patients is provided in Table 1.

**Table 1. Characteristics of patients**

| | Patients, n = 14 | |
|---|---|---|
| | n | % |
| Female gender | 10 | 71.4 |
| Ethnic group, | | |
| Chinese | 8 | 57.1 |
| Malay | 3 | 21.4 |
| Indian | 2 | 14.3 |
| Others | 1 | 7.1 |
| Median age (range) at flare, yrs | 37.6 | 23.8 - 53.9 |
| Median duration (range) of SLE at flare, yrs | 6.1 | 0.0 - 17.8 |
| SLEDAI ≥ 4 | 13 | 92.9 |
| SLEDAI Median (range) score at flare | 11.5 | 2 - 31 |

Among the study patients, tear samples were collected at various times before, during and after flares, forming 3 groups of patients as illustrated in Figure 1. Thus a total of 107 samples from subjects and 20 samples from controls were analysed. The organ involvement of subjects at flare was determined and is shown in Table 2.

**Table 2. Organ involvement of patients at flare**

| Patient | Gender | Ethnicity | Age at flare (yr) | SLEDAI score | BILAG global score | GEN | MUC | NEU | MSK | CAR | VAS | REN | HAE |
|---|---|---|---|---|---|---|---|---|---|---|---|---|---|
| 1 | M | M | 54 | 10 | 13 | E | B | E | D | C | E | A | D |
| 2 | F | I | 44 | 11 | 8 | E | B | E | C | E | E | B | C |
| 3 | F | C | 38 | 5 | 4 | E | D | E | C | E | E | E | B |
| 4 | M | I | 32 | 12 | 10 | D | E | E | D | D | E | A | C |
| 5 | F | C | 58 | 9 | 11 | C | B | E | B | C | C | C | C |
| 6 | F | C | 52 | 6 | 10 | B | B | E | B | E | E | E | C |
| 7 | F | M | 36 | 31 | 25 | B | B | B | B | C | D | A | B |
| | | | | | | | | | | | | | |
| 8 | F | C | 32 | 8 | 8 | C | C | E | C | C | E | B | C |
| 9 | M | C | 51 | 20 | 19 | B | C | E | C | C | B | A | C |
| 10 | F | C | 24 | 22 | 14 | E | C | E | E | E | B | A | C |
| 11 | F | C | 50 | 2 | 11 | C | E | A | D | D | D | D | C |
| 12 | F | M | 36 | 16 | 25 | A | B | E | C | D | D | A | B |
| 13 | F | C | 37 | 12 | 10 | B | B | E | B | E | E | E | C |
| 14 | M | O | 37 | 14 | 15 | B | B | E | B | C | C | B | C |

### METHODS

Briefly, specimens were collected serially in SLE patients (1) at and after a flare, or (2) when quiescent but likely to subsequently flare. iTRAQ sample preparation was followed by two dimensional nano-LC-nano-ESI-mass spectroscopic analysis to identify possible biomarkers in these specimens.

### Subjects

We studied 2 groups of patients with flares:
(1) Subjects with specimens before, during and after flares - up to 8 time points.
(2) Subjects with specimens during and after flares - up to 5 time points.
For each group we also included a specimen several months removed from the time of the flare to act as a control, i.e. when their disease is not active.

### Instruments/ Databases

A brief description of MEXSLEDAI can be found in Guzman J, et al., J Rheumatol 1992 Oct;19(10): 1551-8, incorporated herein by reference. This is a modification of the SLEDAI so that laboratory tests are not included, and was used because the intent of the study was to compare these novel biomarkers with the usual biomarkers of disease activity which are included in the SLEDAI. Different weights are given to various clinical manifestations, some clinical manifestations were added (fatigue, lymphopenia) as per Uribe AG, et al., J Rheumatol 2004 Oct; 31(10): 1934-40, incorporated herein by reference.

### Sample preparation

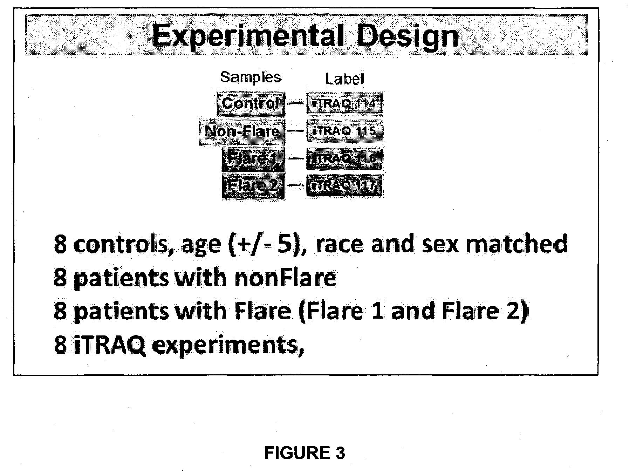

Samples were prepared as described in Lei Zhou et al., Journal of Proteomics 2012; 75: 3877-85. Schirmer strips were cut into small pieces and soaked in 150 µL of 50 mM ammonium bicarbonate solution containing protease inhibitor (Halt protease inhibitor cocktail, Thermoscientific, IL, USA) for 3 hours to elute tear proteins. The protein concentration was measured by colorimetric protein assay (Bio-Rad) using bovine serum album (BSA) as standard. Quantitative proteomics using isobaric tags for relative and absolute quantitation (iTRAQ) technology coupled with 1 D-nanoLC-MS/MS was used to identify potential tear protein biomarkers. The experimental design using iTRAQ relative quantitative proteomics technology is illustrated in Figure 3. Briefly, 50 µg of tear proteins were reconstituted in 50mM ammonium bicarbonate and reduced by Tris-(2-carboxyethyl) phosphine (TCEP). After 1.5 h of incubation at 60 °C, the cysteine residue was blocked by the addition of methyl methanethiosulfonate (MMTS) at room temperature for 20 min. The protein sample was then digested with trypsin (trypsin: protein ratio = 1:50) for 16 h at 37 °C. The samples were subsequently labeled with iTRAQ reagent (AB SCIEX, Framingham,, MA, USA). The samples were then combined and analyzed by one dimensional nanoLC-MS/MS.

### One dimensional nanoLC-MS/MS proteomic analysis

The LC-MS/MS was performed using Dionex UltiMate 3000 (Dionex/Thermo Fisher Scientific, Waltham, MA, USA) coupled with the AB Sciex Triple TOF 5600. For the one dimension separation of peptides, sample was first loaded onto the trap column (Acclaim PepMap 75 mm x 2 cm C18 3 µm x 100 Å by Dionex/Thermo Fisher Scientific, Waltham, MA, USA) for 5 minutes, at a flow rate of 5 µl/min. The flow was then directed in line with the Acclaim PepMap RSLC column 75 mm x 50 cm C18 2µm x 100 A (Dionex/Thermo Fisher Scientific, Waltham, MA, USA) at a flow rate of 0.3 µl/min which is connected to the spray tip (PicoTip Emitter Silica Tip™ by New Objective, Woburn, MA, USA). The total step gradient time was set at 104 minutes. Mobile phases A (0.1% FA, 2% ACN in water) and B (0.1% FA, 2% water in ACN) were used to create the separation gradient for eluting peptides in the column at an increasing concentration of solvent B as follows: 5% to 30% for 69 minutes; 30% to 40% for 27 minutes; 40% to 60% for 7 minutes followed by 60% to 95% for 1 minute.

For IDA (Information dependent acquisition) experiment, all data were acquired from the Triple TOF MS using Analyst TF 1.5 software by AB Sciex in the Information-dependent acquisition (IDA) mode. Peptide profiling was performed using a mass range of 350 to 1250 Da followed by a MS/MS product ion scan from 100 to 1500 Da with the abundance threshold set at more than 120 cps. The accumulation time for ions was set at 50 ms. Target ions were excluded from the scan for 12 s after being detected and former ions were excluded from the scan after one repetition. The IDA advanced 'rolling collision energy (CE)' option was required to automatically ramp up the CE value in the collision cell as the m/z value was increased. A maximum of 30 spectra were collected from candidate ions per cycle.

### Sample preparation for validation

Schirmer strips were cut into small pieces and soaked in 150 µL of 50 mM ammonium bicarbonate solution containing protease inhibitor (Halt protease inhibitor cocktail, Thermoscientific, IL, USA) for 3 hours to elute tear proteins The protein concentration was measured by colorimetric protein assay (Bio-Rad) using bovine serum album (BSA) as standard. 25 µg of tear proteins were reconstituted in 50 mM ammonium bicarbonate and reduced by Tris-(2-carboxyethyl) phosphine (TCEP). After 1.5 h of incubation at 60 °C, the cysteine residue was blocked by the addition of iodoacetamide (IAA) at room temperature for 20 min. The protein sample was then digested with trypsin (trypsin : protein ratio = 1:50) for 16 h at 37 °C. The samples were then desalted using ultra micro spin column (The Nest Group, MA, USA).

25 µg of digested samples were reconstituted in 12 µl of loading buffer (0.1% formic acid, 2% acetonitrile in water) and 250 fmol/µl of DMBT1, LDHA, LDHB, HSPA1B and HSPB1 and 2.5 pmol/µl of ENO1 isotope-standards were spiked into the sample solution to give a final concentration of 50 fmol/µl and 500 fmol/µl respectively.

### One dimensional nanoLC-MS/MS proteomic analysis for validation

RP separation was employed as described in Lei Zhou et al., Progress in Ret and Eye Res, 2012; Nov 31(6): 527-550, using Ultimate 3000 nanoLC system (Dionex, Thermo Fisher Scientific, MA, USA) coupled with AB Sciex 5600 triple TOF (AB Sciex, Framingham, MA, USA) for the analysis. A 15 cm x 75 µm i.d. packed with Acclaim PepMap RSLC C18 column was employed (Dionex, Thermo Fisher Scientific, MA, USA). This column was connected to a spray tip (New Objectives, Woburn, MA), which was directly coupled with the nano-spray interface into AB Sciex 5600 tripleTOF mass spectrometer. Samples were loaded onto a trap column (Acclaim PepMap 100 C18, 2 cm x 75 µm i.d., Dionex, Thermo Fisher Scientific, MA, USA) at a flow rate of 5 µL/min. After a 5 min wash with loading buffer (2/98 v/v of ACN/water with 0.1% formic acid), the system was switched into line with the C18 analytical capillary column. A linear gradient of mobile phase B (2/98 v/v of water/ACN with 0.1% formic acid) from 7% to 30% was run for 34 minutes at flow rate of 300 nL/min was utilized for this analysis.

Third generation Nanospray Source was installed and other instrumentation settings were as follows: lonspray Voltage Floating (ISVF) = 2400 V, curtain gas (CUR) = 30, Ion source gas 1 (GS1) = 12, Interface Heater Temperature (IHT) = 125, Declustering potential (DP) = 100 V, Nebuliser current (NC) = 3 for nitrogen gas. Targeted MRM approach was employed with data acquired using TOF MS + Hi Sensitivity product ion with Analyst TF 1.6 software (AB Sciex, Framingham, MA, USA). TOF-MS scan (experiment 1) parameters were set as follows: 0.25 sec TOF MS accumulation time in the mass range of 350-1250 Da followed by a table of respective peptide ions parameter shown in Table 3.

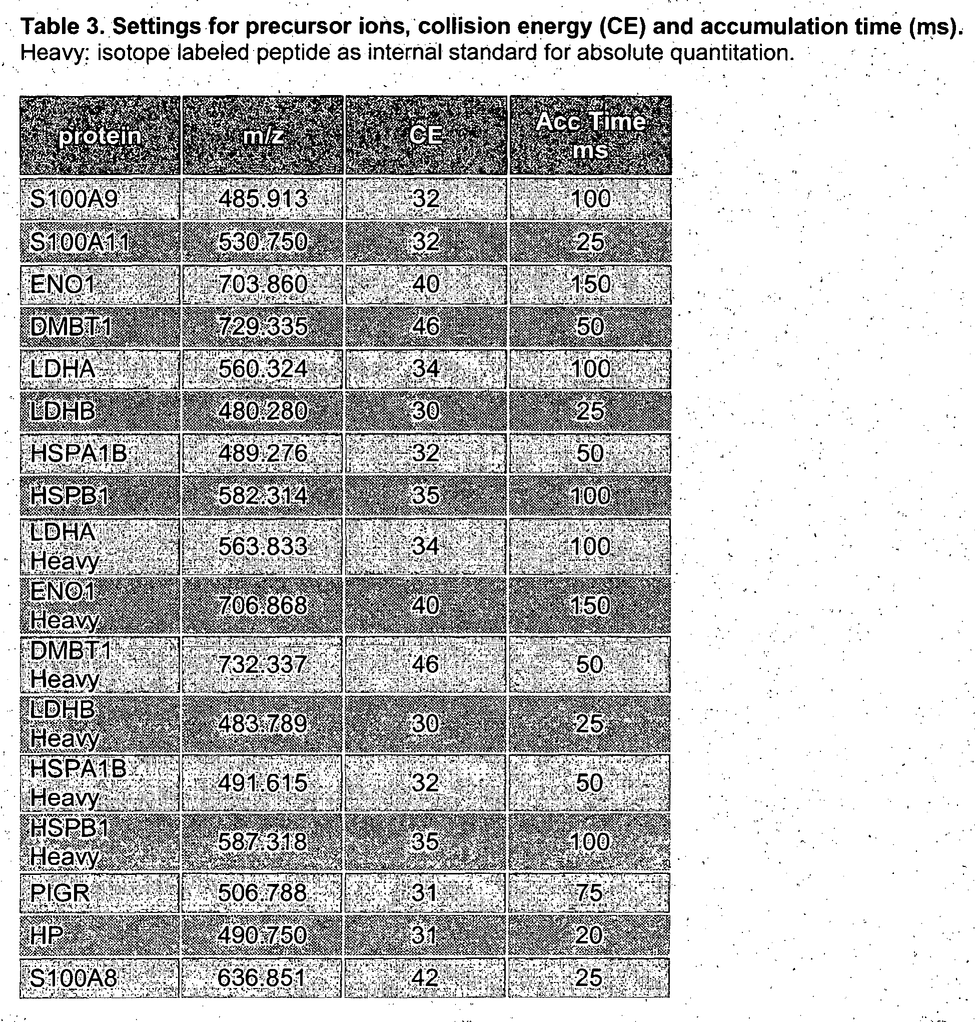

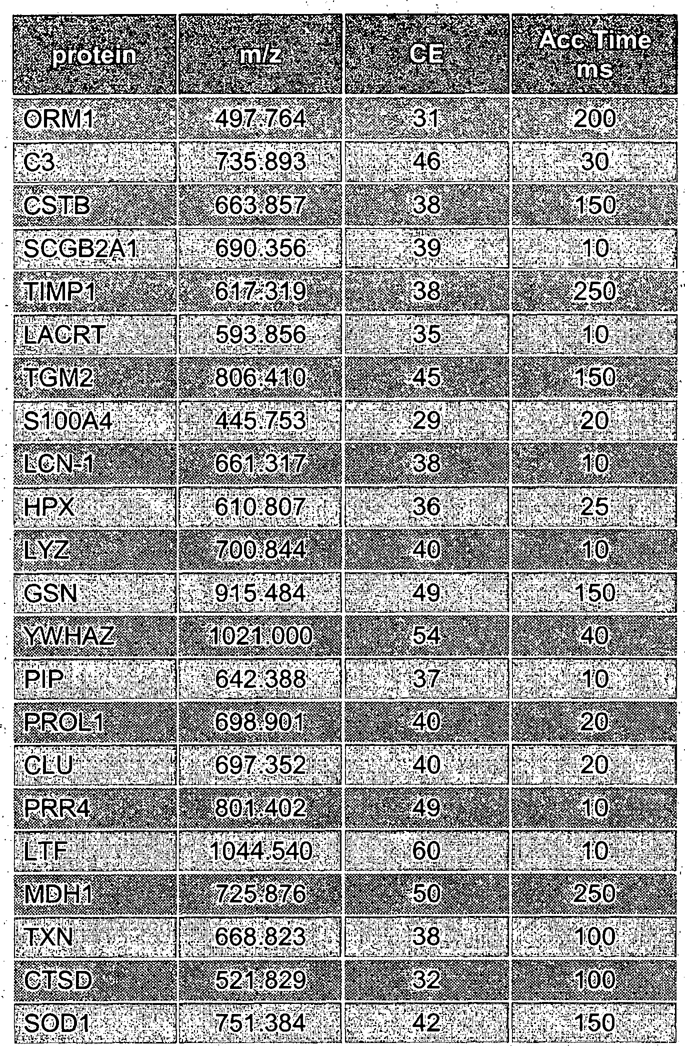

The mass range was set at 100∼1500 Da.

1 µl of spiked sample was injected into Ultimate 3000 nanoLC system (Dionex, Thermo Fisher Scientific, MA, USA) coupled with AB Sciex 5600 triple TOF (AB Sciex, Framingham, MA, USA) for the analysis. 1µl of Global Control digested tear sample (2.083 µg/µl), spiked with the same concentration as the analysed samples, was injected before every 4 sample-injection.

### Statistical Analyses

We hypothesised that tear biomarker levels would correlate with flares of SLE measured using the SLEDAI/ physician global assessment. We studied this using generalised estimating equation (GEE) models with levels of each biomarker as the outcome variable. Biomarkers identified as predictors of SLE flares in the above analysis were also studied as a combined outcome variable using various combinations.

### Statistical approach

- Studied at each individual protein and combinations of proteins.

The Stats package used for data analysis was STATA v12 (Stata Corp, College Station, TX, USA).

### RESULTS

### Biomarker identification

In the first stage, iTRAQ was combined with 1D nanoLC-MS/MS to screen for potential tear biomarker candidates for SLE. Eight patients with flares (each patient has two flare states, Flare 1 and Flare 2, Figure 3) as compared with 8 SLE patients without flares and 8 age, gender and ethnicity matched controls.

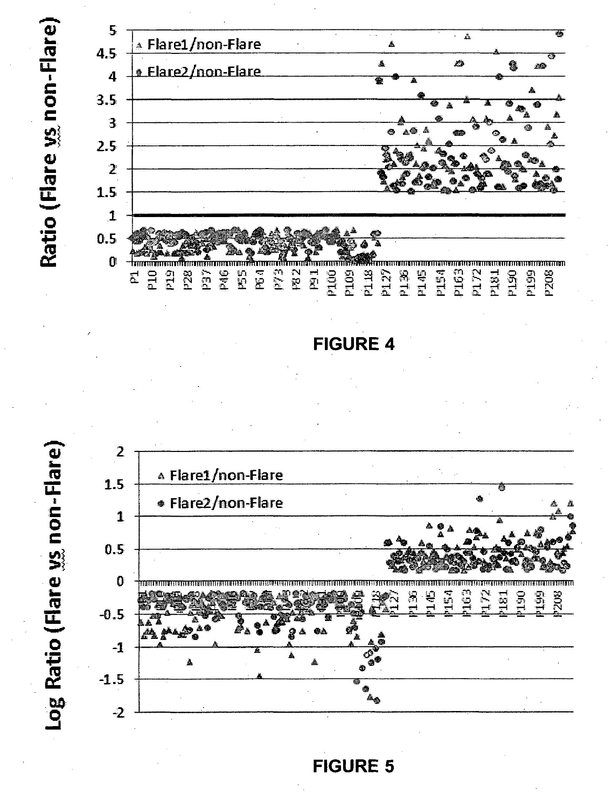

In total, 1580 tear proteins were identified [False Discovery Rate (FDR) < 1%] after combing eight iTRAQ experiments. Among them, 1365 proteins were quantifiable. The expression levels of the biomarker candidates were compared as a ratio of Flare 1 and Flare 2 vs non-Flare average from 8 patients (Figure 4), and as a log ratio (Figure 5). Flare and non-flare states can be distinguished using a tear protein profile.

If the cutoffs for up-regulated or down-regulated were defined as > 1.5 or < 0.67 when Flare compared to non-Flare, 97 proteins and 123 were found to be either up-regulated or down-regulated in the Flare group as compared to the non-Flare group. Further analysis identified biomarkers alpha-Enolase (ENO1), Deleted in Brain Tumour 1 (DMBT1), Lactate dehydrogenase A (LDHA), Lactate dehydrogenase B (LDHB), Heat Shock 70kDa Protein 1B (HSPA1B) and Heat Shock 27kDa Protein 1 (HSPB1) as promising biomarkers.

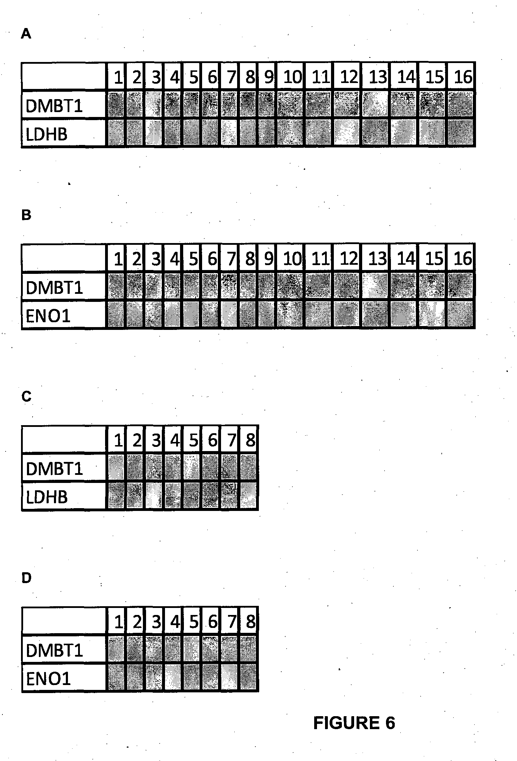

The best down-regulated protein was DMBT1. Taking a flare (8 patients with 2 samples each -at and 2 weeks after a flare) versus Non-Flare ratio < 0.67, 14 out of 16 flares (87.5%) were identified correctly. The best up-regulated protein is LDHB. Using a ratio of < 0.77, 15 out of 16 or 93.85% were correct (Figure 6). With the ratio at > 1.5, 11/16 (68.8%) were correctly identified. For a ratio > 1.3, 12/16 (75.0%) were correct. However, the combination of 2 proteins in a panel can increase the accuracy of diagnosis (Figure 6).

The six best tear protein biomarker candidates (DMBT1, ENO1, LDHA, LDHB, HSPA1B and HSPB1) were selected for subsequent validation in a longitudinal study.

### Validation

In the validation stage, High-resolution Multiple Reaction Monitoring (HR-MRM) and isotope labeled peptide standards were used to perform absolute quantitation of six tear proteins. The validation results showed that 13 out of 14 patients have at least one protein (from the 6-protein panel) matched with the expected profile which increased levels or decreased levels of proteins in flare as compared to pre-flare or post-flare. Among them, DMBT1 showed the expected profile in 12 out of 14 patients (Figure 7).

Singly, ENO1, DMBT1 and LDHB levels are significantly differentially expressed in SLE subjects during a flare compared with after a flare (Tables 4 and 5).

**Table 4 - Description of the 6 proteins and paired comparisons**

| Protein | Median value Pre-Flare (IQR) (N=7) | Median value at Flare (IQR) (N=14) | Median value Post-Flare (IQR) (N=10) | p-value pre vs Flare (N=7)* | p-value post vs Flare (N=10)* | p-value Friedman test (N=3) |
|---|---|---|---|---|---|---|
| HSPB1 | 57.94 (39.11-115.84) | 38.43 (32.79-81.47) | 55.70 (42.43-70.07) | 0.6121 | 0.1394 | 0.5637 |
| HSPA1B | 18.84 (15.12-43.65) | 25.43 (15.32-39.26) | 30.34 (17.04-41.41) | 0.8658 | 0.0745 | 0.5637 |
| LDHA | 7.50 (3.41-9.52) | 6.24 (5.14-8.95) | 5.96 (3.82-7.61) | 0.7353 | 0.9594 | 0.5637 |
| LDHB | 3.19 (2.21-5.37) | 2.91 (0.98-4.41) | 2.82 (1.05-3.76) | 0.7353 | 0.0593 | 0.0833 |
| DMBT1 | 20.48 (16.44 -39.02) | 9.06 (5.12-19.35) | 11.29 (6.51-16.96) | 0.2367 | **0.0284** | 0.5637 |
| ENO1 | 2.59 (2.09-4.83) | 2.60 (1.02-4.78) | 3.11 (1.43-4.56) | 0.8658 | **0.0218** | 0.5637 |

| | | | | | | |
|---|---|---|---|---|---|---|
| *Wilcoxon signed-rank test | | | | | | |

**Table 5 - Marker of Flares identified using Generalised Estimating Equations ***

| Protein | Coef. | Std. Error | p-value |
|---|---|---|---|
| HSPB1 | -0.0013 | 0.002 | 0.424 |
| HSPA1B | -0.004 | 0.005 | 0.404 |
| LDHA | -0.023 | 0.021 | 0.265 |
| **LDHB** | **-0.069** | **0.028** | **0.015** |
| **DMBT1** | **0.006** | **0.003** | **0.058** |
| ENO1 | -0.039 | 0.030 | 0.188 |

| | | | |
|---|---|---|---|
| *No adjustment for covariates because of small sample size | | | |

We have identified protein biomarkers in tear samples from SLE patients that can be used in the clinical setting to indicate the flare status of the patient.

### LIST OF REFERENCES

Abu-Sharaka M, et al: Mortality studies in systemic lupus erythematosus: results from a single centre I. Causes of death. J Rheumatol 1995; 22: 1259-64.
Ehrenstein MR, et al: The occurrence, nature and distribution of flares in a cohort of patients with systemic lupus erythematosus: a rheumatological view. Br J Rheumatol 1995; 34(3): 257-60.
Guzman J, et al: Measurement of disease activity in systemic lupus erythematosus. Prospective validation of 3 clinical indices. J Rheumatol 1992 Oct; 19(10): 1551-8.
Lee LG, et al: A Low-Cost, High-Performance System for Fluorescence Lateral Flow Assays. Biosensors 2013; 3: 360-373.
Lei Zhou et al: In depth analysis of the human tear proteome. Journal of Proteomics 2012; 75: 3877-85.
Lei Zhou et al: Tear analysis in ocular surface disease. Progress in Ret and Eye Res 2012; Nov 31(6): 527-550.
Mirzayan MJ, et al: Prognostic parameters for flare in systemic lupus erythematosus. Rheumatology (Oxford) 2000; 39(12): 1316-9.
Petri M, et al: Morbidity of systemic lupus erythematosus: role of race and socioeconomic status. Am J Med 1991; 91: 345-54.
Petri M, et al: Definition, incidence, and clinical description of flare in systemic lupus erythematosus. A prospective cohort study. Arthritis Rheum 1991; Aug 34(8): 937-44.
Sambrook and Russell, Molecular Cloning: A Laboratory Manual, Cold Springs Harbor Laboratory, New York (2001).
Uribe AG, et al: The Systemic Lupus Activity Measure-revised, the Mexican Systemic Lupus Erythematosus Disease Activity Index (SLEDAI), and a modified SLEDAI-2K are adequate instruments to measure disease activity in systemic lupus erythematosus. J Rheumatol 2004 Oct; 31(10): 1934-40.
Zhou L, et al. Identification of tear fluid biomarkers in dry eye syndrome using iTRAQ quantitative proteomics. J Proteome Res 2009; 8: 4889-905.

## Claims

1. An *in vitro* method of diagnosing or monitoring the flare status of SLE in a subject, comprising screening a fluid test sample from the subject for the presence of at least one biomarker differentially expressed in a flare state compared to a reference sample, wherein the at least one biomarker is selected from the group comprising Enolase 1 (ENO1), ), Deleted in Brain Tumour 1 (DMBT1), Lactate dehydrogenase A (LDHA), Lactate dehydrogenase B (LDHB), Heat Shock 70kDa Protein 1B (HSPA1B) and Heat Shock 27kDa Protein 1 (HSPB1); and
diagnosing or monitoring the status of SLE in the subject based on the expression of the at least one biomarker.

2. The method according to claim 1, wherein the reference sample represents the biomarker expression profile of a normal subject or an SLE subject in a quiescent or flare state.

3. The method according to claim 1, wherein the status of SLE in a subject is a state of incipient flare of disease activity.

4. The method according to claim 1, wherein the expression of the at least one biomarker is detected using tandem Mass Spectrometry, immunoassay, or a nucleic acid technology.

5. The method according to claim 4, wherein the immunoassay is selected from the group comprising western blot, dot blot, enzyme-linked immunosorbent assay (ELISA) and lateral flow assay (LFA).

6. The method according to claim 4, wherein the nucleic acid technology further comprises hybridization or amplification in a quantitative real-time polymerase chain reaction.

7. The method according to claim 4, wherein detecting expression comprises using at least one primer or probe set to detect the expression of the at least one biomarker.

8. The method according to claim 1, wherein the at least one biomarker comprises DMBT1.

9. The method according to claim 1, wherein the at least one biomarker comprises DMBT1 and ENO1, or DMBT1 and LDHB.

10. The method according to claim 1, wherein the at least one biomarker is an expressed protein.

11. The method according to claim 1, wherein the test sample is a tear or saliva sample.

## Patentansprüche

1. *In vitro*-Verfahren zum Diagnostizieren oder Überwachen des Status eines Schubs von SLE in einem Subjekt, umfassend das Screening einer fluiden Testprobe von einem Subjekt auf das Vorhandensein von mindestens einem Biomarker, der in einem Schubzustand im Vergleich zu einer Referenzprobe differentiell exprimiert wird, wobei der mindestens eine Biomarker ausgewählt ist aus der Gruppe, umfassend Enolase 1 (ENO1), Deletiert im Hirntumor 1 (DMBT1), Lactatdehydrogenase A (LDHA), Lactatdehydrogenase B (LDHB), Hitzeschock-70kDa-Protein 1B (HSPA1B) und Hitzeschock 27kDa-Protein 1 (HSPB1); und
Diagnostizieren oder Überwachen des Status des Ausbruchs von SLE in dem Subjekt auf der Basis der Expression des mindestens einen Biomarkers.

2. Verfahren nach Anspruch 1, wobei die Referenzprobe das Profil der Expression des Biomarkers eines normalen Subjekts oder eines SLE-Subjekts in einem asymptomatischen oder Schubzustand repräsentiert.

3. Verfahren nach Anspruch 1, wobei der Status des SLE in einem Subjekt ein Zustand eines beginnenden Schubs der Krankheitsaktivität ist.

4. Verfahren nach Anspruch 1, wobei die Expression des mindestens einen Biomarkers unter Anwendung von Tandem-Massenspektrometrie, Immunoassay oder einer Nucleinsäure-Technologie detektiert wird.

5. Verfahren nach Anspruch 4, wobei der Immunoassay ausgewählt ist aus der Gruppe, umfassend Western Blot, Dot Blot, enzymgekoppelter Immunadsorptionstest (ELISA) und Lateral Flow Assay (LFA).

6. Verfahren nach Anspruch 4, wobei die Nucleinsäure-Technologie ferner eine Hybridisierung oder Amplifikation in einer quantitativen Echtzeit-Polymerase-Kettenreaktion umfasst.

7. Verfahren nach Anspruch 4, wobei das Detektieren von Expression das Verwenden mindestens eines Primer- oder Sonden-Satzes umfasst, um die Expression des mindestens einen Biomarkers zu detektieren.

8. Verfahren nach Anspruch 1, wobei der mindestens eine Biomarker DMBT1 umfasst.

9. Verfahren nach Anspruch 1, wobei der mindestens eine Biomarker DMBT1 und ENO1 oder DMBT1 und LDHB umfasst.

10. Verfahren nach Anspruch 1, wobei der mindestens eine Biomarker ein exprimiertes Protein ist.

11. Verfahren nach Anspruch 1, wobei die Testprobe eine Tränen- oder Speichelprobe ist.

## Revendications

1. Procédé *in vitro* de diagnostic ou de surveillance de l'état de poussée du lupus érythémateux systémique - SLE - chez un sujet, comprenant le dépistage d'un échantillon de test fluide issu du sujet quant à la présence d'au moins un biomarqueur qui est exprimé de façon différentielle dans un état de poussée par comparaison avec un échantillon de référence, dans lequel l'au moins un biomarqueur est sélectionné parmi le groupe qui comprend l'énolase 1 (ENO1), supprimé dans tumeur cervicale 1 (DMBT1), déshydrogénase de lactate A (LDHA), déshydrogénase de lactate B (LDHB), protéine 70kDa de choc thermique 1B (HSPA1B) et protéine 27kDa de choc thermique 1 (HSPB1) ; et
le diagnostic ou la surveillance de l'état du SLE chez le sujet sur la base de l'expression de l'au moins un biomarqueur.

2. Procédé selon la revendication 1, dans lequel l'échantillon de référence représente le profil d'expression du biomarqueur d'un sujet normal ou d'un sujet atteint de SLE dans un état quiescent ou de poussée.

3. Procédé selon la revendication 1, dans lequel l'état du SLE chez un sujet est un état de poussée naissante de l'activité de la maladie.

4. Procédé selon la revendication 1, dans lequel l'expression de l'au moins un biomarqueur est détectée en utilisant une spectrométrie de masse en tandem, un dosage immunologique ou une technologie par acide(s) nucléique(s).

5. Procédé selon la revendication 4, dans lequel le dosage immunologique est sélectionné parmi le groupe qui comprend le transfert de type western, le transfert en point, le procédé immunoenzymétique à double détermination d'anticorps (ELISA) et le dosage à écoulement latéral (LFA).

6. Procédé selon la revendication 4, dans lequel la technologie par acide(s) nucléique(s) comprend en outre une hybridation ou une amplification selon une réaction en chaîne par polymérase temps réel quantitative.

7. Procédé selon la revendication 4, dans lequel la détection de l'expression comprend l'utilisation d'au moins un amorceur ou une sonde configuré(e) de manière à ce qu'il/elle détecte l'expression de l'au moins un biomarqueur.

8. Procédé selon la revendication 1, dans lequel l'au moins un biomarqueur comprend DMBT1.

9. Procédé selon la revendication 1, dans lequel l'au moins un biomarqueur comprend DMBT1 et ENO1, ou DMBT1 et LDHB.

10. Procédé selon la revendication 1, dans lequel l'au moins un biomarqueur est une protéine exprimée.

11. Procédé selon la revendication 1, dans lequel l'échantillon de test est un échantillon de larme ou de salive.


### SEQUENCE LISTING

<110> Singapore Health services Pte Ltd
<120> Identification of Novel Biomarkers of Flares of Systemic Lupus
   Erythematosus
<130> FP7087
<150> 201306892-9
   <151> 2013-09-11
<160> 12
<170> PatentIn version 3.5
<210> 1
   <211> 434
   <212> PRT
   <213> Homo sapiens
<400> 1
   Met Ser Ile Leu Lys Ile His Ala Arg Glu Ile Phe Asp Ser Arg Gly
   1               5                   10                  15
   Asn Pro Thr Val Glu Val Asp Leu Phe Thr Ser Lys Gly Leu Phe Arg
               20                  25                  30
   Ala Ala Val Pro Ser Gly Ala Ser Thr Gly Ile Tyr Glu Ala Leu Glu
           35                  40                  45
   Leu Arg Asp Asn Asp Lys Thr Arg Tyr Met Gly Lys Gly Val Ser Lys
       50                  55                  60
   Ala Val Glu His Ile Asn Lys Thr Ile Ala Pro Ala Leu Val Ser Lys
   65                  70                  75                  80
   Lys Leu Asn Val Thr Glu Gln Glu Lys Ile Asp Lys Leu Met Ile Glu
                   85                  90                  95
   Met Asp Gly Thr Glu Asn Lys Ser Lys Phe Gly Ala Asn Ala Ile Leu
               100                 105                 110
   Gly Val Ser Leu Ala Val Cys Lys Ala Gly Ala Val Glu Lys Gly Val
           115                 120                 125
   Pro Leu Tyr Arg His Ile Ala Asp Leu Ala Gly Asn Ser Glu Val Ile
       130                 135                 140
   Leu Pro Val Pro Ala Phe Asn Val Ile Asn Gly Gly Ser His Ala Gly
   145                 150                 155                 160
   Asn Lys Leu Ala Met Gln Glu Phe Met Ile Leu Pro Val Gly Ala Ala
                   165                 170                 175
   Asn Phe Arg Glu Ala Met Arg Ile Gly Ala Glu Val Tyr His Asn Leu
   180 185 190
   Lys Asn Val Ile Lys Glu Lys Tyr Gly Lys Asp Ala Thr Asn Val Gly
   195 200 205
   Asp Glu Gly Gly Phe Ala Pro Asn Ile Leu Glu Asn Lys Glu Gly Leu
   210 215 220
   Glu Leu Leu Lys Thr Ala Ile Gly Lys Ala Gly Tyr Thr Asp Lys Val
   225 230 235 240
   Val Ile Gly Met Asp Val Ala Ala Ser Glu Phe Phe Arg Ser Gly Lys
   245 250 255
   Tyr Asp Leu Asp Phe Lys Ser Pro Asp Asp Pro Ser Arg Tyr Ile Ser
   260 265 270
   Pro Asp Gln Leu Ala Asp Leu Tyr Lys Ser Phe Ile Lys Asp Tyr Pro
   275 280 285
   Val Val Ser Ile Glu Asp Pro Phe Asp Gln Asp Asp Trp Gly Ala Trp
   290 295 300
   Gln Lys Phe Thr Ala Ser Ala Gly Ile Gln Val Val Gly Asp Asp Leu
   305 310 315 320
   Thr Val Thr Asn Pro Lys Arg Ile Ala Lys Ala Val Asn Glu Lys Ser
   325 330 335
   Cys Asn Cys Leu Leu Leu Lys Val Asn Gln Ile Gly Ser Val Thr Glu
   340 345 350
   Ser Leu Gln Ala Cys Lys Leu Ala Gln Ala Asn Gly Trp Gly Val Met
   355 360 365
   Val Ser His Arg Ser Gly Glu Thr Glu Asp Thr Phe Ile Ala Asp Leu
   370 375 380
   Val Val Gly Leu Cys Thr Gly Gln Ile Lys Thr Gly Ala Pro Cys Arg
   385 390 395 400
   Ser Glu Arg Leu Ala Lys Tyr Asn Gln Leu Leu Arg Ile Glu Glu Glu
   405 410 415
   Leu Gly Ser Lys Ala Lys Phe Ala Gly Arg Asn Phe Arg Asn Pro Leu
   420 425 430
   Ala Lys
<210> 2
   <211> 1755
   <212> DNA
   <213> Homo sapiens
<400> 2
   acggagatct cgccggcttt acgttcacct cggtgtctgc agcaccctcc gcttcctctc     60
   ctaggcgacg agacccagtg gctagaagtt caccatgtct attctcaaga tccatgccag    120
   ggagatcttt gactctcgcg ggaatcccac tgttgaggtt gatctcttca cctcaaaagg    180
   tctcttcaga gctgctgtgc ccagtggtgc ttcaactggt atctatgagg ccctagagct    240
   ccgggacaat gataagactc gctatatggg gaagggtgtc tcaaaggctg ttgagcacat    300
   caataaaact attgcgcctg ccctggttag caagaaactg aacgtcacag aacaagagaa    360
   gattgacaaa ctgatgatcg agatggatgg aacagaaaat aaatctaagt ttggtgcgaa    420
   cgccattctg gggggtgtcc ttgccgtctg caaagctggt gccgttgaga agggggtccc    480
   cctgtaccgc cacatcgctg acttggctgg caactctgaa gtcatcctgc cagtcccggc    540
   gttcaatgtc atcaatggcg gttctcatgc tggcaacaag ctggccatgc aggagttcat    600
   gatcctccca gtcggtgcag caaacttcag ggaagccatg cgcattggag cagaggttta    660
   ccacaacctg aagaatgtca tcaaggagaa atatgggaaa gatgccacca atgtggggga    720
   tgaaggcggg tttgctccca acatcctgga gaataaagaa ggcctggagc tgctgaagac    780
   tgctattggg aaagctggct acactgataa ggtggtcatc ggcatggacg tagcggcctc    840
   cgagttcttc aggtctggga agtatgacct ggacttcaag tctcccgatg accccagcag    900
   gtacatctcg cctgaccagc tggctgacct gtacaagtcc ttcatcaagg actacccagt    960
   ggtgtctatc gaagatccct ttgaccagga tgactgggga gcttggcaga agttcacagc   1020
   cagtgcagga atccaggtag tgggggatga tctcacagtg accaacccaa agaggatcgc   1080
   caaggccgtg aacgagaagt cctgcaactg cctcctgctc aaagtcaacc agattggctc   1140
   cgtgaccgag tctcttcagg cgtgcaagct ggcccaggcc aatggttggg gcgtcatggt   1200
   gtctcatcgt tcgggggaga ctgaagatac cttcatcgct gacctggttg tggggctgtg   1260
   cactgggcag atcaagactg gtgccccttg ccgatctgag cgcttggcca agtacaacca   1320
   gctcctcaga attgaagagg agctgggcag caaggctaag tttgccggca ggaacttcag   1380
   aaaccccttg gccaagtaag ctgtgggcag gcaagccttc ggtcacctgt tggctacaca   1440
   gacccctccc ctcgtgtcag ctcaggcagc tcgaggcccc cgaccaacac ttgcaggggt   1500
   ccctgctagt tagcgcccca ccgccgtgga gttcgtaccg cttccttaga acttctacag   1560
   aagccaagct ccctggagcc ctgttggcag ctctagcttt tgcagtcgtg taatgggccc   1620
   aagtcattgt ttttctcgcc tcactttcca ccaagtgtct agagtcatgt gagcctcgtg   1680
   tcatctccgg ggtggccaca ggctagatcc ccggtggttt tgtgctcaaa ataaaaagcc   1740
   tcagtgaccc atgag                                                    1755
<210> 3
   <211> 2413
   <212> PRT
   <213> Homo sapiens
<400> 3
   Met Gly Ile Ser Thr Val Ile Leu Glu Met Cys Leu Leu Trp Gly Gln
   1               5                   10                  15
   Val Leu Ser Thr Gly Gly Trp Ile Pro Arg Thr Thr Asp Tyr Ala Ser
               20                  25                  30
   Leu Ile Pro Ser Glu Val Pro Leu Asp Pro Thr Val Ala Glu Gly Ser
           35                  40                  45
   Pro Phe Pro Ser Glu Ser Thr Leu Glu Ser Thr Val Ala Glu Gly Ser
       50                  55                  60
   Pro Ile Ser Leu Glu Ser Thr Leu Glu Ser Thr Val Ala Glu Gly Ser
   65                  70                  75                  80
   Leu Ile Pro Ser Glu Ser Thr Leu Glu Ser Thr Val Ala Glu Gly Ser
                   85                  90                  95
   Asp Ser Gly Leu Ala Leu Arg Leu Val Asn Gly Asp Gly Arg Cys Gln
               100                 105                 110
   Gly Arg Val Glu Ile Leu Tyr Arg Gly Ser Trp Gly Thr Val Cys Asp
           115                 120                 125
   Asp Ser Trp Asp Thr Asn Asp Ala Asn Val Val Cys Arg Gln Leu Gly
       130                 135                 140
   Cys Gly Trp Ala Met Ser Ala Pro Gly Asn Ala Trp Phe Gly Gln Gly
   145                 150                 155                 160
   Ser Gly Pro Ile Ala Leu Asp Asp Val Arg Cys Ser Gly His Glu Ser
                   165                 170                 175
   Tyr Leu Trp Ser Cys Pro His Asn Gly Trp Leu Ser His Asn Cys Gly
               180                 185                 190
   His Gly Glu Asp Ala Gly Val Ile Cys Ser Ala Ala Gln Pro Gln Ser
           195                 200                 205
   Thr Leu Arg Pro Glu Ser Trp Pro Val Arg Ile Ser Pro Pro Val Pro
       210                 215                 220
   Thr Glu Gly Ser Glu Ser Ser Leu Ala Leu Arg Leu Val Asn Gly Gly
   225                 230                 235                 240
   Asp Arg Cys Arg Gly Arg Val Glu Val Leu Tyr Arg Gly Ser Trp Gly
                   245                 250                 255
   Thr Val Cys Asp Asp Tyr Trp Asp Thr Asn Asp Ala Asn Val Val Cys
               260                 265                 270
   Arg Gln Leu Gly Cys Gly Trp Ala Met Ser Ala Pro Gly Asn Ala Gln
           275                 280                 285
   Phe Gly Gln Gly Ser Gly Pro Ile Val Leu Asp Asp Val Arg Cys Ser
       290                 295                 300
   Gly His Glu Ser Tyr Leu Trp Ser Cys Pro His Asn Gly Trp Leu Thr
   305                 310                 315                 320
   His Asn Cys Gly His Ser Glu Asp Ala Gly Val Ile Cys Ser Ala Pro
                   325                 330                 335
   Gln Ser Arg Pro Thr Pro Ser Pro Asp Thr Trp Pro Thr Ser His Ala
               340                 345                 350
   Ser Thr Ala Gly Pro Glu Ser Ser Leu Ala Leu Arg Leu Val Asn Gly
           355                 360                 365
   Gly Asp Arg Cys Gln Gly Arg Val Glu Val Leu Tyr Arg Gly Ser Trp
       370                 375                 380
   Gly Thr Val Cys Asp Asp Ser Trp Asp Thr Ser Asp Ala Asn Val Val
   385                 390                 395                 400
   Cys Arg Gln Leu Gly Cys Gly Trp Ala Thr Ser Ala Pro Gly Asn Ala
                   405                 410                 415
   Arg Phe Gly Gln Gly Ser Gly Pro Ile Val Leu Asp Asp Val Arg Cys
               420                 425                 430
   Ser Gly Tyr Glu Ser Tyr Leu Trp Ser Cys Pro His Asn Gly Trp Leu
           435                 440                 445
   Ser His Asn Cys Gln His Ser Glu Asp Ala Gly Val Ile Cys Ser Ala
       450                 455                 460
   Ala His Ser Trp Ser Thr Pro Ser Pro Asp Thr Leu Pro Thr Ile Thr
   465                 470                 475                 480
   Leu Pro Ala Ser Thr Val Gly Ser Glu Ser Ser Leu Ala Leu Arg Leu
                   485                 490                 495
   Val Asn Gly Gly Asp Arg Cys Gln Gly Arg Val Glu Val Leu Tyr Arg
               500                 505                 510
   Gly Ser Trp Gly Thr Val Cys Asp Asp Ser Trp Asp Thr Asn Asp Ala
           515                 520                 525
   Asn Val Val Cys Arg Gln Leu Gly Cys Gly Trp Ala Met Leu Ala Pro
       530                 535                 540
   Gly Asn Ala Arg Phe Gly Gln Gly Ser Gly Pro Ile Val Leu Asp Asp
   545                 550                 555                 560
   Val Arg Cys Ser Gly Asn Glu Ser Tyr Leu Trp Ser Cys Pro His Asn
                   565                 570                 575
   Gly Trp Leu Ser His Asn Cys Gly His Ser Glu Asp Ala Gly Val Ile
               580                 585                 590
   Cys Ser Gly Pro Glu Ser Ser Leu Ala Leu Arg Leu Val Asn Gly Gly
           595                 600                 605
   Asp Arg Cys Gln Gly Arg Val Glu Val Leu Tyr Arg Gly Ser Trp Gly
       610                 615                 620
   Thr Val Cys Asp Asp Ser Trp Asp Thr Asn Asp Ala Asn Val Val Cys
   625                 630                 635                 640
   Arg Gln Leu Gly Cys Gly Trp Ala Thr Ser Ala Pro Gly Asn Ala Arg
                   645                 650                 655
   Phe Gly Gln Gly Ser Gly Pro Ile Val Leu Asp Asp Val Arg Cys Ser
               660                 665                 670
   Gly His Glu Ser Tyr Leu Trp Ser Cys Pro Asn Asn Gly Trp Leu Ser
           675                 680                 685
   His Asn Cys Gly His His Glu Asp Ala Gly Val Ile Cys Ser Ala Ala
       690                 695                 700
   Gln Ser Arg Ser Thr Pro Arg Pro Asp Thr Leu Ser Thr Ile Thr Leu
   705                 710                 715                 720
   Pro Pro Ser Thr Val Gly Ser Glu Ser Ser Leu Thr Leu Arg Leu Val
                   725                 730                 735
   Asn Gly Ser Asp Arg Cys Gln Gly Arg Val Glu Val Leu Tyr Arg Gly
               740                 745                 750
   Ser Trp Gly Thr Val Cys Asp Asp Ser Trp Asp Thr Asn Asp Ala Asn
           755                 760                 765
   Val Val Cys Arg Gln Leu Gly Cys Gly Trp Ala Thr Ser Ala Pro Gly
       770                 775                 780
   Asn Ala Arg Phe Gly Gln Gly Ser Gly Pro Ile Val Leu Asp Asp Val
   785                 790                 795                 800
   Arg Cys Ser Gly His Glu Ser Tyr Leu Trp Ser Cys Pro His Asn Gly
                   805                 810                 815
   Trp Leu Ser His Asn Cys Gly His His Glu Asp Ala Gly Val Ile Cys
               820                 825                 830
   Ser Val Ser Gln Ser Arg Pro Thr Pro Ser Pro Asp Thr Trp Pro Thr
           835                 840                 845
   Ser His Ala Ser Thr Ala Gly Pro Glu Ser Ser Leu Ala Leu Arg Leu
       850                 855                 860
   Val Asn Gly Gly Asp Arg Cys Gln Gly Arg Val Glu Val Leu Tyr Arg
   865                 870                 875                 880
   Gly Ser Trp Gly Thr Val Cys Asp Asp Ser Trp Asp Thr Ser Asp Ala
                   885                 890                 895
   Asn Val Val Cys Arg Gln Leu Gly Cys Gly Trp Ala Thr Ser Ala Pro
               900                 905                 910
   Gly Asn Ala Arg Phe Gly Gln Gly Ser Gly Pro Ile Val Leu Asp Asp
           915                 920                 925
   Val Arg Cys Ser Gly Tyr Glu Ser Tyr Leu Trp Ser Cys Pro His Asn
       930                 935                 940
   Gly Trp Leu Ser His Asn Cys Gln His Ser Glu Asp Ala Gly Val Ile
   945                 950                 955                 960
   Cys Ser Ala Ala His Ser Trp Ser Thr Pro Ser Pro Asp Thr Leu Pro
                   965                 970                 975
   Thr Ile Thr Leu Pro Ala Ser Thr Val Gly Ser Glu Ser Ser Leu Ala
               980                 985                 990
   Leu Arg Leu Val Asn Gly Gly Asp  Arg Cys Gln Gly Arg  Val Glu Val
           995                 1000                 1005
   Leu Tyr  Gln Gly Ser Trp Gly  Thr Val Cys Asp Asp  Ser Trp Asp
       1010                 1015                 1020
   Thr Asn  Asp Ala Asn Val Val  Cys Arg Gln Leu Gly  Cys Gly Trp
       1025                 1030                 1035
   Ala Met  Ser Ala Pro Gly Asn  Ala Arg Phe Gly Gln  Gly Ser Gly
       1040                 1045                 1050
   Pro Ile  Val Leu Asp Asp Val  Arg Cys Ser Gly His  Glu Ser Tyr
       1055                 1060                 1065
   Leu Trp Ser Cys Pro His Asn Gly Trp Leu Ser His Asn Cys Gly
   1070 1075 1080
   His Ser Glu Asp Ala Gly Val Ile Cys Ser Ala Ser Gln Ser Arg
   1085 1090 1095
   Pro Thr Pro Ser Pro Asp Thr Trp Pro Thr Ser His Ala Ser Thr
   1100 1105 1110
   Ala Gly Ser Glu Ser Ser Leu Ala Leu Arg Leu Val Asn Gly Gly
   1115 1120 1125
   Asp Arg Cys Gln Gly Arg Val Glu Val Leu Tyr Arg Gly Ser Trp
   1130 1135 1140
   Gly Thr Val Cys Asp Asp Tyr Trp Asp Thr Asn Asp Ala Asn Val
   1145 1150 1155
   Val Cys Arg Gln Leu Gly Cys Gly Trp Ala Met Ser Ala Pro Gly
   1160 1165 1170
   Asn Ala Arg Phe Gly Gln Gly Ser Gly Pro Ile Val Leu Asp Asp
   1175 1180 1185
   Val Arg Cys Ser Gly His Glu Ser Tyr Leu Trp Ser Cys Pro His
   1190 1195 1200
   Asn Gly Trp Leu Ser His Asn Cys Gly His His Glu Asp Ala Gly
   1205 1210 1215
   Val Ile Cys Ser Ala Ser Gln Ser Gln Pro Thr Pro Ser Pro Asp
   1220 1225 1230
   Thr Trp Pro Thr Ser His Ala Ser Thr Ala Gly Ser Glu Ser Ser
   1235 1240 1245
   Leu Ala Leu Arg Leu Val Asn Gly Gly Asp Arg Cys Gln Gly Arg
   1250 1255 1260
   Val Glu Val Leu Tyr Arg Gly Ser Trp Gly Thr Val Cys Asp Asp
   1265 1270 1275
   Tyr Trp Asp Thr Asn Asp Ala Asn Val Val Cys Arg Gln Leu Gly
   1280 1285 1290
   Cys Gly Trp Ala Thr Ser Ala Pro Gly Asn Ala Arg Phe Gly Gln
   1295 1300 1305
   Gly Ser Gly Pro Ile Val Leu Asp Asp Val Arg Cys Ser Gly His
   1310 1315 1320
   Glu Ser Tyr Leu Trp Ser Cys Pro His Asn Gly Trp Leu Ser His
   1325 1330 1335
   Asn Cys Gly His His Glu Asp Ala Gly Val Ile Cys Ser Ala Ser
   1340 1345 1350
   Gln Ser Gln Pro Thr Pro Ser Pro Asp Thr Trp Pro Thr Ser His
   1355 1360 1365
   Ala Ser Thr Ala Gly Ser Glu Ser Ser Leu Ala Leu Arg Leu Val
   1370 1375 1380
   Asn Gly Gly Asp Arg Cys Gln Gly Arg Val Glu Val Leu Tyr Arg
   1385 1390 1395
   Gly Ser Trp Gly Thr Val Cys Asp Asp Tyr Trp Asp Thr Asn Asp
   1400 1405 1410
   Ala Asn Val Val Cys Arg Gln Leu Gly Cys Gly Trp Ala Thr Ser
   1415 1420 1425
   Ala Pro Gly Asn Ala Arg Phe Gly Gln Gly Ser Gly Pro Ile Val
   1430 1435 1440
   Leu Asp Asp Val Arg Cys Ser Gly His Glu Ser Tyr Leu Trp Ser
   1445 1450 1455
   Cys Pro His Asn Gly Trp Leu Ser His Asn Cys Gly His His Glu
   1460 1465 1470
   Asp Ala Gly Val Ile Cys Ser Ala Ser Gln Ser Gln Pro Thr Pro
   1475 1480 1485
   Ser Pro Asp Thr Trp Pro Thr Ser Arg Ala Ser Thr Ala Gly Ser
   1490 1495 1500
   Glu Ser Thr Leu Ala Leu Arg Leu Val Asn Gly Gly Asp Arg Cys
   1505 1510 1515
   Arg Gly Arg Val Glu Val Leu Tyr Gln Gly Ser Trp Gly Thr Val
   1520 1525 1530
   Cys Asp Asp Tyr Trp Asp Thr Asn Asp Ala Asn Val Val Cys Arg
   1535 1540 1545
   Gln Leu Gly Cys Gly Trp Ala Met Ser Ala Pro Gly Asn Ala Gln
   1550 1555 1560
   Phe Gly Gln Gly Ser Gly Pro Ile Val Leu Asp Asp Val Arg Cys
   1565 1570 1575
   Ser Gly His Glu Ser Tyr Leu Trp Ser Cys Pro His Asn Gly Trp
   1580 1585 1590
   Leu Ser His Asn Cys Gly His His Glu Asp Ala Gly Val Ile Cys
   1595 1600 1605
   Ser Ala Ala Gln Ser Gln Ser Thr Pro Arg Pro Asp Thr Trp Leu
   1610 1615 1620
   Thr Thr Asn Leu Pro Ala Leu Thr Val Gly Ser Glu Ser Ser Leu
   1625 1630 1635
   Ala Leu Arg Leu Val Asn Gly Gly Asp Arg Cys Arg Gly Arg Val
   1640 1645 1650
   Glu Val Leu Tyr Arg Gly Ser Trp Gly Thr Val Cys Asp Asp Ser
   1655 1660 1665
   Trp Asp Thr Asn Asp Ala Asn Val Val Cys Arg Gln Leu Gly Cys
   1670 1675 1680
   Gly Trp Ala Met Ser Ala Pro Gly Asn Ala Arg Phe Gly Gln Gly
   1685 1690 1695
   Ser Gly Pro Ile Val Leu Asp Asp Val Arg Cys Ser Gly Asn Glu
   1700 1705 1710
   Ser Tyr Leu Trp Ser Cys Pro His Lys Gly Trp Leu Thr His Asn
   1715 1720 1725
   Cys Gly His His Glu Asp Ala Gly Val Ile Cys Ser Ala Thr Gln
   1730 1735 1740
   Ile Asn Ser Thr Thr Thr Asp Trp Trp His Pro Thr Thr Thr Thr
   1745 1750 1755
   Thr Ala Arg Pro Ser Ser Asn Cys Gly Gly Phe Leu Phe Tyr Ala
   1760 1765 1770
   Ser Gly Thr Phe Ser Ser Pro Ser Tyr Pro Ala Tyr Tyr Pro Asn
   1775 1780 1785
   Asn Ala Lys Cys Val Trp Glu Ile Glu Val Asn Ser Gly Tyr Arg
   1790 1795 1800
   Ile Asn Leu Gly Phe Ser Asn Leu Lys Leu Glu Ala His His Asn
   1805 1810 1815
   Cys Ser Phe Asp Tyr Val Glu Ile Phe Asp Gly Ser Leu Asn Ser
   1820 1825 1830
   Ser Leu Leu Leu Gly Lys Ile Cys Asn Asp Thr Arg Gln Ile Phe
   1835 1840 1845
   Thr Ser Ser Tyr Asn Arg Met Thr Ile His Phe Arg Ser Asp Ile
   1850 1855 1860
   Ser Phe Gln Asn Thr Gly Phe Leu Ala Trp Tyr Asn Ser Phe Pro
   1865 1870 1875
   Ser Asp Ala Thr Leu Arg Leu Val Asn Leu Asn Ser Ser Tyr Gly
   1880 1885 1890
   Leu Cys Ala Gly Arg Val Glu Ile Tyr His Gly Gly Thr Trp Gly
   1895 1900 1905
   Thr Val Cys Asp Asp Ser Trp Thr Ile Gln Glu Ala Glu Val Val
   1910 1915 1920
   Cys Arg Gln Leu Gly Cys Gly Arg Ala Val Ser Ala Leu Gly Asn
   1925 1930 1935
   Ala Tyr Phe Gly Ser Gly Ser Gly Pro Ile Thr Leu Asp Asp Val
   1940 1945 1950
   Glu Cys Ser Gly Thr Glu Ser Thr Leu Trp Gln Cys Arg Asn Arg
   1955 1960 1965
   Gly Trp Phe Ser His Asn Cys Asn His Arg Glu Asp Ala Gly Val
   1970 1975 1980
   Ile Cys Ser Gly Asn His Leu Ser Thr Pro Ala Pro Phe Leu Asn
   1985 1990 1995
   Ile Thr Arg Pro Asn Thr Asp Tyr Ser Cys Gly Gly Phe Leu Ser
   2000 2005 2010
   Gln Pro Ser Gly Asp Phe Ser Ser Pro Phe Tyr Pro Gly Asn Tyr
   2015 2020 2025
   Pro Asn Asn Ala Lys Cys Val Trp Asp Ile Glu Val Gln Asn Asn
   2030 2035 2040
   Tyr Arg Val Thr Val Ile Phe Arg Asp Val Gln Leu Glu Gly Gly
   2045 2050 2055
   Cys Asn Tyr Asp Tyr Ile Glu Val Phe Asp Gly Pro Tyr Arg Ser
   2060 2065 2070
   Ser Pro Leu Ile Ala Arg Val Cys Asp Gly Ala Arg Gly Ser Phe
   2075 2080 2085
   Thr Ser Ser Ser Asn Phe Met Ser Ile Arg Phe Ile Ser Asp His
   2090 2095 2100
   Ser Ile Thr Arg Arg Gly Phe Arg Ala Glu Tyr Tyr Ser Ser Pro
   2105 2110 2115
   Ser Asn Asp Ser Thr Asn Leu Leu Cys Leu Pro Asn His Met Gln
   2120 2125 2130
   Ala Ser Val Ser Arg Ser Tyr Leu Gln Ser Leu Gly Phe Ser Ala
   2135 2140 2145
   Ser Asp Leu Val Ile Ser Thr Trp Asn Gly Tyr Tyr Glu Cys Arg
   2150 2155 2160
   Pro Gln Ile Thr Pro Asn Leu Val Ile Phe Thr Ile Pro Tyr Ser
   2165 2170 2175
   Gly Cys Gly Thr Phe Lys Gln Ala Asp Asn Asp Thr Ile Asp Tyr
   2180 2185 2190
   Ser Asn Phe Leu Thr Ala Ala Val Ser Gly Gly Ile Ile Lys Arg
   2195 2200 2205
   Arg Thr Asp Leu Arg Ile His Val Ser Cys Arg Met Leu Gln Asn
   2210 2215 2220
   Thr Trp Val Asp Thr Met Tyr Ile Ala Asn Asp Thr Ile His Val
   2225 2230 2235
   Ala Asn Asn Thr Ile Gln Val Glu Glu Val Gln Tyr Gly Asn Phe
   2240 2245 2250
   Asp Val Asn Ile Ser Phe Tyr Thr Ser Ser Ser Phe Leu Tyr Pro
   2255 2260 2265
   Val Thr Ser Arg Pro Tyr Tyr Val Asp Leu Asn Gln Asp Leu Tyr
   2270 2275 2280
   Val Gln Ala Glu Ile Leu His Ser Asp Ala Val Leu Thr Leu Phe
   2285 2290 2295
   Val Asp Thr Cys Val Ala Ser Pro Tyr Ser Asn Asp Phe Thr Ser
   2300 2305 2310
   Leu Thr Tyr Asp Leu Ile Arg Ser Gly Cys Val Arg Asp Asp Thr
   2315 2320 2325
   Tyr Gly Pro Tyr Ser Ser Pro Ser Leu Arg Ile Ala Arg Phe Arg
   2330 2335 2340
   Phe Arg  Ala Phe His Phe Leu  Asn Arg Phe Pro Ser  Val Tyr Leu
       2345                 2350                 2355
   Arg Cys  Lys Met Val Val Cys  Arg Ala Tyr Asp Pro  Ser Ser Arg
       2360                 2365                 2370
   Cys Tyr  Arg Gly Cys Val Leu  Arg Ser Lys Arg Asp  Val Gly Ser
       2375                 2380                 2385
   Tyr Gln  Glu Lys Val Asp Val  Val Leu Gly Pro Ile  Gln Leu Gln
       2390                 2395                 2400
   Thr Pro  Pro Arg Arg Glu Glu  Glu Pro Arg
       2405                 2410
<210> 4
   <211> 5802
   <212> DNA
   <213> Homo sapiens
<400> 4
   tttatagcag cagcagaaat ataccaccct agaggacaca cctcctttta gctaggtacc 60
   tataaatgtc caggattttc tattcaattg agaagaaccc agcaaaatgg ggatctccac 120
   agtcatcctt gaaatgtgtc ttttatgggg acaagttcta tctacaggtg ggtggatccc 180
   aaggactaca gactacgctt cactgattcc ctcggaggtg cccttggatc aaactgtagc 240
   agaaggttct ccatttccct cggagtcgac cctggagtca actgcagcag aaggttctcc 300
   gatttccttg gagtcaaccc tggagtcaac tgtagcagaa ggttctctga ttccctcaga 360
   gtcaaccctg gagtcaactg tagcagaagg atctgattct ggtttggccc tgaggctggt 420
   gaatggagat ggcaggtgtc agggccgagt ggagatccta taccgaggct cctggggcac 480
   cgtgtgtgat gacagctggg acaccaatga tgccaacgtg gtctgtaggc agctgggttg 540
   tggctgggcc atgtcagctc caggaaatgc ctggtttggc cagggctcag gacccattgc 600
   cctggatgat gtgcgctgct caggacacga atcctacctg tggagctgcc cccacaatgg 660
   ctggctctcc cataactgtg gccatggtga agatgctggt gttatctgct cagctgccca 720
   gcctcagtca acactcaggc cagaaagttg gcctgtcagg atatcaccac ctgtacccac 780
   agaaggatct gaatccagtt tggccctgag gctggtgaat ggaggcgaca ggtgtcgagg 840
   ccgagtggag gtcctatacc gaggctcctg gggcaccgtg tgtgatgact actgggacac 900
   caatgatgcc aatgtggtct gcaggcagct gggctgtggc tgggccatgt cagccccagg 960
   aaatgcccag tttggccagg gctcaggacc cattgtcctg gatgatgtgc gctgctcagg 1020
   acacgagtcc tacctgtgga gctgccccca caatggctgg ctcacccaca actgtggcca 1080
   tagtgaagac gctggtgtca tctgctcagc tccccagtcc cggccgacac ccagcccaga 1140
   tacttggccg acctcacatg catcaacagc aggacctgaa tccagtttgg ccctgaggct 1200
   ggtgaatgga ggtgacaggt gtcagggccg agtggaggtc ctataccgag gctcctgggg 1260
   caccgtgtgt gatgatagct gggacaccag tgacgccaat gtggtctgcc ggcagctggg 1320
   ctgtggctgg gccacgtcag ccccaggaaa tgcccggttt ggccagggtt caggacccat 1380
   tgtcctggat gacgtgcgct gctcaggcta tgagtcctac ctgtggagct gcccccacaa 1440
   tggctggctc tcccataact gtcagcacag tgaagacgct ggtgtcatct gctcagctgc 1500
   ccactcctgg tcgacgccca gtccagacac attgccgacc atcaccttgc ctgcatcgac 1560
   agtaggatct gaatccagtt tggccctgag gctggtgaat ggaggtgaca ggtgtcaggg 1620
   ccgagtggag gtcctatacc aaggctcctg gggcaccgtg tgcgatgaca gctgggacac 1680
   caatgatgcc aatgtcgtct gcaggcaacc gggctgtggc tgggccatgt cagccccagg 1740
   aaatgcccgg tttggtcagg gctcaggacc cattgtcctg gatgatgtgc gctgctcagg 1800
   acacgagtct tacccgtgga gctgccccca caatggctgg ctctcccaca actgtggcca 1860
   tagtgaagac gctggtgtca tctgctcagc ttcccagtcc cggccaacac ctagtccaga 1920
   cacttggcca acctcacatg catcaacagc aggatctgaa tccagtttgg ccctgaggct 1980
   ggtgaatgga ggtgacaggt gtcagggccg agtggaggtc ctataccgag gctcctgggg 2040
   caccgtgtgt gatgactact gggacaccaa tgatgccaat gtggtttgca ggcagctggg 2100
   ctgtggctgg gccatgtcag ccccaggaaa tgcccggttt ggccagggtt caggacccat 2160
   tgtcctggat gatgtgcgct gctcaggaca tgagtcctat ctgtggagct gcccccacaa 2220
   tggctggctc tcccacaact gtggccatca tgaagacgct ggtgtcatct gctcagcttc 2280
   ccagtcccag ccgacaccca gcccagacac ttggccaacc tcacatgcat caacagcagg 2340
   atctgaatcc agtttggccc tgaggctggt gaatggaggt gacaggtgtc agggccgagt 2400
   ggaggtccta taccgaggct cctggggcac cgtgtgtgat gactactggg acaccaatga 2460
   tgccaatgtg gtttgcaggc agctgggctg tggctgggcc acgtcagccc caggaaatgc 2520
   ccggtttggc cagggttcag gacccattgt cctggatgat gtgcgctgct caggacatga 2580
   gtcctatctg tggagctgcc cccacaatgg ctggctctcc cacaactgtg gccatcatga 2640
   agacgctggt gtcatctgct cagcttccca gtcccagccg acacccagcc cagacacttg 2700
   gccaacctct cgtgcatcaa cagcaggatc tgaatccact ttggccctga gactggtgaa 2760
   tggaggtgac aggtgtcgag gccgagtgga ggtcctatac caaggctcct ggggcaccgt 2820
   gtgtgatgac tactgggaca ccaatgatgc caacgtggtc tgcaggcagc tgggctgtgg 2880
   ctgggccatg tcagccccag gaaatgccca gtttggccag ggctcaggac ccattgtcct 2940
   ggatgatgtg cgctgctcag gacacgagtc ttacctgtgg agctgccccc acaatggctg 3000
   gctctcccac aactgtggcc atcatgaaga tgctggtgtc atctgctcag ctgctcagtc 3060
   ccagtcaacg cccaggccag atacttggct gaccaccaac ttaccggcat tgacagtagg 3120
   atctgaatcc agtttggctc tgaggctggt gaatggaggt gacaggtgtc gaggccgagt 3180
   ggaggtcctg tatcgaggct cctggggaac cgtgtgtgat gacagctggg acaccaatga 3240
   tgccaatgtg gtctgcaggc agctgggctg tggctgggcc atgtcggccc caggaaatgc 3300
   ccggtttggc cagggctcag gacccattgt cctggatgat gtgcgctgct cagggaatga 3360
   gtcctacctg tggagctgcc cccacaaagg ctggctcacc cacaactgtg gccatcacga 3420
   agacgctggt gtcatctgct cagccaccca aataaattct actacgacag attggtggca 3480
   tccaacaact acaaccactg caagaccctc ttcaaattgt ggtggcttct tattctatgc 3540
   cagtgggaca ttctccagcc catcctaccc tgcatactac cccaacaatg ctaagtgtgt 3600
   ttgggaaata gaagtgaatt ctggttatcg cataaacctg ggcttcagta atctgaaatt 3660
   ggaggcacac cataactgca gttttgatta tgttgaaatc tttgatggat cattgaatag 3720
   cagtctcctg ctggggaaaa tctgtaatga taccaggcaa atatttacat cttcttacaa 3780
   ccgaatgacc attcactttc gaagtgacat cagtttccaa aacactggct ttttggcttg 3840
   gtataactcc ttcccaagcg atgccacctt gaggttggtc aatttaaatt catcctatgg 3900
   tctatgtgcc gggcgtgtag aaatttacca tggtggcacc tgggggacag tttgtgatga 3960
   ctcctggacc attcaggaag ctgaggtggt ctgcagacag ctagggtgtg gacgtgcagt 4020
   ttcagccctt ggaaatgcat attttggctc tggctctggc cccatcaccc tggacgatgt 4080
   agagtgctca gggacggaat ccactctctg gcagtgccgg aaccgaggct ggttctccca 4140
   caactgtaat catcgtgaag atgctggtgt catctgctca ggaaaccatc tatcgacacc 4200
   tgctcctttt ctcaacatca cccgtccaaa cacagattat tcctgcggag gcttcctatc 4260
   ccaaccatca ggggactttt ccagcccatt ctatcccggg aactatccaa acaatgccaa 4320
   gtgtgtgtgg gacattgagg tgcaaaacaa ctaccgtgtg actgtgatct tcagagatgt 4380
   ccagcttgaa ggtggctgca actatgatta tattgaagtt ttcgatggcc cctaccgcag 4440
   ttcccctctc attgctcgag tttgtgatgg ggccagaggc tccttcactt cttcctccaa 4500
   cttcatgtcc attcgcttca tcagtgacca cagcatcaca aggagagggt tccgggctga 4560
   gtactactcc agtccctcca atgacagcac caacctgctc tgtctgccaa atcacatgca 4620
   agccagtgtg agcaggagct atctccaatc cttgggcttt tctgccagtg accttgtcat 4680
   ttccacctgg aatggatact acgagtgtcg gccccagata acgccgaacc tggtgatatt 4740
   cacaattccc tactcaggct gcggcacctt caagcaggca gacaatgaca ccatcgacta 4800
   ttccaacttc ctcacagcag ctgtctcagg tggcatcatc aagaggagga cagacctccg 4860
   tattcacgtc agctgcagaa tgcttcagaa cacctgggtc gacaccatgt acattgctaa 4920
   tgacaccatc cacgttgcta ataacaccat ccaggtcgag gaagtccagt atggcaattt 4980
   tgacgtgaac atttcctttt atacttcctc atctttcttg tatcctgtga ccagccgccc 5040
   ttactacgtg gacctgaacc aggacttgta cgttcaggct gaaatcctcc attctgatgc 5100
   tgtactgacc ttgtttgtgg acacctgcgt ggcatcacca tactccaatg acttcacgtc 5160
   tttgacttat gatctaatcc ggagtggatg cgtgagggat gacacctacg gaccctactc 5220
   ctcgccgtct cttcgcattg cccgcttccg gttcagggcc ttccacttcc tgaaccgctt 5280
   cccctccgtg tacctgcgtt gtaaaatggt ggtgtgcaga gcgtatgacc cctcttcccg 5340
   ctgctaccga ggctgtgtgt tgaggtcgaa gagggatgtg ggctcctacc aggaaaaggt    5400
   ggacgtcgtc ctgggtccca tccagctgca gaccccccca cgccgagaag aggagcctcg    5460
   gtaggtggtc gctctcagac cccactgtcc accggggcgc agacccctga ctcggggact    5520
   tgggatgttc ctcttggtgt catattccaa ctcagattga gccctacatt gtgctgcacc    5580
   tggtcatacg gagttgaatc agacctggtt cccgcctccc ccaaggctca tggtccttgg    5640
   aggacccgtt gcagggcgag gtcaagagag ttctgacctg gatggcccat agacctgacg    5700
   tcccagaatc catgcttctc atctgcaaaa tgaaaatgtc aatacttact tcttagcact    5760
   gttgagaggg ttacttacat aaaggaattt tggtgaaact gc                       5802
<210> 5
   <211> 334
   <212> PRT
   <213> Homo sapiens
<400> 5
   Met Ala Thr Leu Lys Glu Lys Leu Ile Ala Pro Val Ala Glu Glu Glu
   1 5 10 15
   Ala Thr Val Pro Asn Asn Lys Ile Thr Val Val Gly Val Gly Gln Val
   20 25 30
   Gly Met Ala Cys Ala Ile Ser Ile Leu Gly Lys Ser Leu Ala Asp Glu
   35 40 45
   Leu Ala Leu Val Asp Val Leu Glu Asp Lys Leu Lys Gly Glu Met Met
   50 55 60
   Asp Leu Gln His Gly Ser Leu Phe Leu Gln Thr Pro Lys Ile Val Ala
   65 70 75 80
   Asp Lys Asp Tyr Ser Val Thr Ala Asn Ser Lys Ile Val Val Val Thr
   85 90 95
   Ala Gly Val Arg Gln Gln Glu Gly Glu Ser Arg Leu Asn Leu Val Gln
   100 105 110
   Arg Asn Val Asn Val Phe Lys Phe Ile Ile Pro Gln Ile Val Lys Tyr
   115 120 125
   Ser Pro Asp Cys Ile Ile Ile Val Val Ser Asn Pro Val Asp Ile Leu
   130 135 140
   Thr Tyr Val Thr Trp Lys Leu Ser Gly Leu Pro Lys His Arg Val Ile
   145 150 155 160
   Gly Ser Gly Cys Asn Leu Asp Ser Ala Arg Phe Arg Tyr Leu Met Ala
   165 170 175
   Glu Lys Leu Gly Ile His Pro Ser Ser Cys His Gly Trp Ile Leu Gly
   180 185 190
   Glu His Gly Asp Ser Ser Val Ala Val Trp Ser Gly Val Asn Val Ala
   195 200 205
   Gly Val Ser Leu Gln Glu Leu Asn Pro Glu Met Gly Thr Asp Asn Asp
   210 215 220
   Ser Glu Asn Trp Lys Glu Val His Lys Met Val Val Glu Ser Ala Tyr
   225 230 235 240
   Glu Val Ile Lys Leu Lys Gly Tyr Thr Asn Trp Ala Ile Gly Leu Ser
   245 250 255
   Val Ala Asp Leu Ile Glu Ser Met Leu Lys Asn Leu Ser Arg Ile His
   260 265 270
   Pro Val Ser Thr Met Val Lys Gly Met Tyr Gly Ile Glu Asn Glu Val
   275 280 285
   Phe Leu Ser Leu Pro Cys Ile Leu Asn Ala Arg Gly Leu Thr Ser Val
   290 295 300
   Ile Asn Gln Lys Leu Lys Asp Asp Glu Val Ala Gln Leu Lys Lys Ser
   305 310 315 320
   Ala Asp Thr Leu Trp Asp Ile Gln Lys Asp Leu Lys Asp Leu
   325 330
<210> 6
   <211> 1318
   <212> DNA
   <213> Homo sapiens
<400> 6
   ggagacgcac gcagctgact ttgtcttctc cgcacgactg ttacagaggt ctccagagcc      60
   ttctctctcc tgtgcaaaat ggcaactctt aaggaaaaac tcattgcacc agttgcggaa     120
   gaagaggcaa cagttccaaa caataagatc actgtagtgg gtgttggaca agttggtatg     180
   gcgtgtgcta tcagcattct gggaaagtct ctggctgatg aacttgctct tgtggatgtt     240
   ttggaagata agcttaaagg agaaatgatg gatctgcagc atgggagctt atttcttcag     300
   acacctaaaa ttgtggcaga taaagattat tctgtgaccg ccaattctaa gattgtagtg     360
   gtaactgcag gagtccgtca gcaagaaggg gagagtcggc tcaatctggt gcagagaaat     420
   gttaatgtct tcaaattcat tattcctcag atcgtcaagt acagtcctga ttgcatcata     480
   attgtggttt ccaacccagt ggacattctt acgtatgtta cctggaaact aagtggatta     540
   cccaaacacc gcgtgattgg aagtggatgt aatctggatt ctgctagatt tcgctacctt     600
   atggctgaaa aacttggcat tcatcccagc agctgccatg gatggatttt gggggaacat     660
   ggcgactcaa gtgtggctgt gtggagtggt gtgaatgtgg caggtgtttc tctccaggaa     720
   ttgaatccag aaatgggaac tgacaatgat agtgaaaatt ggaaggaagt gcataagatg     780
   gtggttgaaa gtgcctatga agtcatcaag ctaaaaggat ataccaactg ggctattgga     840
   ttaagtgtgg ctgatcttat tgaatccatg ttgaaaaatc tatccaggat tcatcccgtg     900
   tcaacaatgg taaaggggat gtatggcatt gagaatgaag tcttcctgag ccttccatgt     960
   atcctcaatg cccggggatt aaccagcgtt atcaaccaga agctaaagga tgatgaggtt    1020
   gctcagctca agaaaagtgc agataccctg tgggacatcc agaaggacct aaaagacctg    1080
   tgactagtga gctctaggct gtagaaattt aaaaactaca atgtgattaa ctcgagcctt    1140
   tagttttcat ccatgtacat ggatcacagt ttgctttgat cttcttcaat atgtgaattt    1200
   gggctcacag aatcaaagcc tatgcttggt ttaatgcttg caatctgagc tcttgaacaa    1260
   ataaaattaa ctattgtagt gtgaaaaaaa aaaaaaaaaa aaaaaaaaaa aaaaaaaa       1318
<210> 7
   <211> 332
   <212> PRT
   <213> Homo sapiens
<400> 7
<210> 8
   <211> 1031
   <212> DNA
   <213> Homo sapiens
<400> 8
   attcccgatt ccttttggtt ccaagtccaa tatggcaact ctaaaggatc agctgattta 60
   taatcttcta aaggaagaac agacccccca gaataagatt acagttgttg gggttggtgc 120
   tgttggcatg gcctgtgcca tcagtatctt aatgaaggac ttggcagatg aacttgctct 180
   tgttgatgtc atcgaagaca aattgaaggg agagatgatg gatctccaac atggcagcct 240
   tttccttaga acaccaaaga ttgtctctgg caaagactat aatgtaactg caaactccaa 300
   gctggtcatt atcacggctg gggcacgtca gcaagaggga gaaagccgtc ttaatttggt 360
   ccagcgtaac gtgaacatat ttaaattcat cattcctaat gttgtaaaat acagcccgaa 420
   ctgcaagttg cttattgttt caaatccagt ggatatcttg acctacgtgg cttggaagat 480
   aagtggtttt cccaaaaacc gtgttattgg aagtggttgc aatctggatt cagcccgatt 540
   ccgttacctg atgggggaaa ggctgggagt tcacccatta agctgtcatg ggtgggtcct 600
   tggggaacat ggagattcca gtgtgcctgt atggagtgga atgaatgttg ctggtgtctc 660
   tctgaagact ctgcacccag atttagggac tgataaagat aaggaacagt ggaaagaggt 720
   tcacaagcag gtggttgaga gtgcttatga ggtgatcaaa ctcaaaggct acacatcctg 780
   ggctattgga ctctctgtag cagatttggc agagagtata atgaagaatc ttaggcgggt 840
   gcacccagtt tccaccatga ttaagggtct ttacggaata aaggatgatg tcttccttag 900
   tgttccttgc attttgggac agaatggaat ctcagacctt gtgaaggtga ctctgacttc 960
   tgaggaagag gcccgtttga agaagagtgc agatacactt tgggggatcc aaaaggagct 1020
   gcaattttaa a 1031
<210> 9
   <211> 641
   <212> PRT
   <213> Homo sapiens
<400> 9
   Met Ala Lys Ala Ala Ala Ile Gly Ile Asp Leu Gly Thr Thr Tyr Ser
   1               5                   10                  15
   Cys Val Gly Val Phe Gln His Gly Lys Val Glu Ile Ile Ala Asn Asp
               20                  25                  30
   Gln Gly Asn Arg Thr Thr Pro Ser Tyr Val Ala Phe Thr Asp Thr Glu
           35                  40                  45
   Arg Leu Ile Gly Asp Ala Ala Lys Asn Gln Val Ala Leu Asn Pro Gln
       50                  55                  60
   Asn Thr Val Phe Asp Ala Lys Arg Leu Ile Gly Arg Lys Phe Gly Asp
   65                  70                  75                  80
   Pro Val Val Gln Ser Asp Met Lys His Trp Pro Phe Gln Val Ile Asn
                   85                  90                  95
   Asp Gly Asp Lys Pro Lys Val Gln Val Ser Tyr Lys Gly Glu Thr Lys
               100                 105                 110
   Ala Phe Tyr Pro Glu Glu Ile Ser Ser Met Val Leu Thr Lys Met Lys
           115                 120                 125
   Glu Ile Ala Glu Ala Tyr Leu Gly Tyr Pro Val Thr Asn Ala Val Ile
       130                 135                 140
   Thr Val Pro Ala Tyr Phe Asn Asp Ser Gln Arg Gln Ala Thr Lys Asp
   145                 150                 155                 160
   Ala Gly Val Ile Ala Gly Leu Asn Val Leu Arg Ile Ile Asn Glu Pro
                   165                 170                 175
   Thr Ala Ala Ala Ile Ala Tyr Gly Leu Asp Arg Thr Gly Lys Gly Glu
               180                 185                 190
   Arg Asn Val Leu Ile Phe Asp Leu Gly Gly Gly Thr Phe Asp Val Ser
           195                 200                 205
   Ile Leu Thr Ile Asp Asp Gly Ile Phe Glu Val Lys Ala Thr Ala Gly
       210                 215                 220
   Asp Thr His Leu Gly Gly Glu Asp Phe Asp Asn Arg Leu Val Asn His
   225                 230                 235                 240
   Phe Val Glu Glu Phe Lys Arg Lys His Lys Lys Asp Ile Ser Gln Asn
                   245                 250                 255
   Lys Arg Ala Val Arg Arg Leu Arg Thr Ala Cys Glu Arg Ala Lys Arg
               260                 265                 270
   Thr Leu Ser Ser Ser Thr Gln Ala Ser Leu Glu Ile Asp Ser Leu Phe
           275                 280                 285
   Glu Gly Ile Asp Phe Tyr Thr Ser Ile Thr Arg Ala Arg Phe Glu Glu
       290                 295                 300
   Leu Cys Ser Asp Leu Phe Arg Ser Thr Leu Glu Pro Val Glu Lys Ala
   305                 310                 315                 320
   Leu Arg Asp Ala Lys Leu Asp Lys Ala Gln Ile His Asp Leu Val Leu
                   325                 330                 335
   Val Gly Gly Ser Thr Arg Ile Pro Lys Val Gln Lys Leu Leu Gln Asp
               340                 345                 350
   Phe Phe Asn Gly Arg Asp Leu Asn Lys Ser Ile Asn Pro Asp Glu Ala
           355                 360                 365
   Val Ala Tyr Gly Ala Ala Val Gln Ala Ala Ile Leu Met Gly Asp Lys
       370                 375                 380
   Ser Glu Asn Val Gln Asp Leu Leu Leu Leu Asp Val Ala Pro Leu Ser
   385                 390                 395                 400
   Leu Gly Leu Glu Thr Ala Gly Gly Val Met Thr Ala Leu Ile Lys Arg
                   405                 410                 415
   Asn Ser Thr Ile Pro Thr Lys Gln Thr Gln Ile Phe Thr Thr Tyr Ser
               420                 425                 430
   Asp Asn Gln Pro Gly Val Leu Ile Gln Val Tyr Glu Gly Glu Arg Ala
           435                 440                 445
   Met Thr Lys Asp Asn Asn Leu Leu Gly Arg Phe Glu Leu Ser Gly Ile
       450                 455                 460
   Pro Pro Ala Pro Arg Gly Val Pro Gln Ile Glu Val Thr Phe Asp Ile
   465                 470                 475                 480
   Asp Ala Asn Gly Ile Leu Asn Val Thr Ala Thr Asp Lys Ser Thr Gly
                   485                 490                 495
   Lys Ala Asn Lys Ile Thr Ile Thr Asn Asp Lys Gly Arg Leu Ser Lys
               500                 505                 510
   Glu Glu Ile Glu Arg Met Val Gln Glu Ala Glu Lys Tyr Lys Ala Glu
           515                 520                 525
   Asp Glu Val Gln Arg Glu Arg Val Ser Ala Lys Asn Ala Leu Glu Ser
       530                 535                 540
   Tyr Ala Phe Asn Met Lys Ser Ala Val Glu Asp Glu Gly Leu Lys Gly
   545                 550                 555                 560
   Lys Ile Ser Glu Ala Asp Lys Lys Lys Val Leu Asp Lys Cys Gln Glu
                   565                 570                 575
   Val Ile Ser Trp Leu Asp Ala Asn Thr Leu Ala Glu Lys Asp Glu Phe
               580                 585                 590
   Glu His Lys Arg Lys Glu Leu Glu Gln Val Cys Asn Pro Ile Ile Ser
           595                 600                 605
   Gly Leu Tyr Gln Gly Ala Gly Gly Pro Gly Pro Gly Gly Phe Gly Ala
       610                 615                 620
   Gln Gly Pro Lys Gly Gly Ser Gly Ser Gly Pro Thr Ile Glu Glu Val
   625                 630                 635                 640
   Asp
<210> 10
   <211> 2379
   <212> DNA
   <213> Homo sapiens
<400> 10
   cctttttcga gagtgactcc cgttgtccca aggcttccca gagcgaacct gtgcggctgc      60
   aggcaccggc gcgtcgagtt tccggcgtcc ggaaggaccg agctcttctc gcggatccag     120
   tgttccgttt ccagccccca atctcagagc ggagccgaca gagagcaggg aaccggcatg     180
   gccaaagccg cggcgatcgg catcgacctg ggcaccacct actcctgcgt gggggtgttc 240
   caacacggca aggtggagat catcgccaac gaccagggca accgcaccac ccccagctac 300
   gtggccttca cggacaccga gcggctcatc ggggatgcgg ccaagaacca ggtggcgctg 360
   aacccgcaga acaccgtgtt tgacgcgaag cggctgatcg gccgcaagtt cggcgacccg 420
   gtggtgcagt cggacatgaa gcactggcct ttccaggtga tcaacgacgg agacaagccc 480
   aaggtgcagg tgagctacaa gggggagacc aaggcattct accccgagga gatctcgtcc 540
   atggtgctga ccaagatgaa ggagatcgcc gaggcgtacc tgggctaccc ggtgaccaac 600
   gcggtgatca ccgtgccggc ctacttcaac gactcgcagc gccaggccac caaggatgcg 660
   ggtgtgatcg cggggctcaa cgtgctgcgg atcatcaacg agcccacggc cgccgccatc 720
   gcctacggcc tggacagaac gggcaagggg gagcgcaacg tgctcatctt tgacctgggc 780
   gggggcacct tcgacgtgtc catcctgacg atcgacgacg gcatcttcga ggtgaaggcc 840
   acggccgggg acacccacct gggtggggag gactttgaca acaggctggt gaaccacttc 900
   gtggaggagt tcaagagaaa acacaagaag gacatcagcc agaacaagcg agccgtgagg 960
   cggctgcgca ccgcctgcga gagggccaag aggaccctgt cgtccagcac ccaggccagc 1020
   ctggagatcg actccctgtt tgagggcatc gacttctaca cgtccatcac cagggcgagg 1080
   ttcgaggagc tgtgctccga cctgttccga agcaccctgg agcccgtgga gaaggctctg 1140
   cgcgacgcca agctggacaa ggcccagatt cacgacctgg tcctggtcgg gggctccacc 1200
   cgcatcccca aggtgcagaa gctgctgcag gacttcttca acgggcgcga cctgaacaag 1260
   agcatcaacc ccgacgaggc tgtggcctac ggggcggcgg tgcaggcggc catcctgatg 1320
   ggggacaagt ccgagaacgt gcaggacctg ctgctgctgg acgtggctcc cctgtcgctg 1380
   gggctggaga cggccggagg cgtgatgact gccctgatca agcgcaactc caccatcccc 1440
   accaagcaga cgcagatctt caccacctac tccgacaacc aacccggggt gctgatccag 1500
   gtgtacgagg gcgagagggc catgacgaaa gacaacaatc tgttggggcg cttcgagctg 1560
   agcggcatcc ctccggcccc caggggcgtg ccccagatcg aggtgacctt cgacatcgat 1620
   gccaacggca tcctgaacgt cacggccacg gacaagagca ccggcaaggc caacaagatc 1680
   accatcacca acgacaaggg ccgcctgagc aaggaggaga tcgagcgcat ggtgcaggag 1740
   gcggagaagt acaaagcgga ggacgaggtg cagcgcgaga gggtgtcagc caagaacgcc 1800
   ctggagtcct acgccttcaa catgaagagc gccgtggagg atgaggggct caagggcaag 1860
   atcagcgagg cggacaagaa gaaggttctg gacaagtgtc aagaggtcat ctcgtggctg 1920
   gacgccaaca ccttggccga gaaggacgag tttgagcaca agaggaagga gctggagcag 1980
   gtgtgtaacc ccatcatcag cggactgtac cagggtgccg gtggtcccgg gcctgggggc 2040
   ttcggggctc agggtcccaa gggagggtct gggtcaggcc ccaccattga ggaggtggat 2100
   taggggcctt tccaagattg ctgtttttgt tttggagctt caagactttg catttcctag 2160
   tatttctgtt tgtcagttct caatttcctg tgtttgcaat gttgaaattt tttggtgaag 2220
   tactgaactt gctttttttc cggtttctac atgcagagat gaatttatac tgccatctta 2280
   cgactatttc ttctttttaa tacacttaac tcaggccatt ttttaagttg gttacttcaa 2340
   agtaaataaa ctttaaaatt caaaaaaaaa aaaaaaaaa 2379
<210> 11
   <211> 205
   <212> PRT
   <213> Homo sapiens
<400> 11
<210> 12
   <211> 789
   <212> DNA
   <213> Homo sapiens
<400> 12
   gcccagcgcc ccgcactttt ctgagcagac gtccagagca gagtcagcca gcatgaccga      60
   gcgccgcgtc cccttctcgc tcctgcgggg ccccagctgg gaccccttcc gcgactggta     120
   cccgcatagc cgcctcttcg accaggcctt cgggctgccc cggctgccgg aggagtggtc     180
   gcagtggtta ggcggcagca gctggccagg ctacgtgcgc cccctgcccc ccgccgccat     240
   cgagagcccc gcagtggccg cgcccgccta cagccgcgcg ctcagccggc aactcagcag     300
   cggggtctcg gagatccggc acactgcgga ccgctggcgc gtgtccctgg atgtcaacca     360
   cttcgccccg gacgagctga cggtcaagac caaggatggc gtggtggaga tcaccggcaa     420
   gcacgaggag cggcaggacg agcatggcta catctcccgg tgcttcacgc ggaaatacac     480
   gctgcccccc ggtgtggacc ccacccaagt ttcctcctcc ctgtcccctg agggcacact     540
   gaccgtggag gcccccatgc ccaagctagc cacgcagtcc aacgagatca ccatcccagt     600
   caccttcgag tcgcgggccc agcttggggg cccagaagct gcaaaatccg atgagactgc     660
   cgccaagtaa agccttagcc cggatgccca cccctgctgc cgccactggc tgtgcctccc     720
   ccgccacctg tgtgttcttt tgatacattt atcttctgtt tttctcaaat aaagttcaaa     780
   gcaaccacc                                                             789